# EUROPEAN PATENT SPECIFICATION

(11) **EP 4 480 382 B1**
(45) Date of publication and mention of the grant of the patent: **01.04.2026**
(21) Application number: 23756604.7
(22) Date of filing: 14.02.2023
(51) Int. Cl.: A61B 1/00, A61B 1/12, A61B 1/24, A61C 9/00, A61B 5/00, A61C 19/04, H04N 1/00, H04N 1/32

(54) **ELECTRONIC DEVICE AND METHOD FOR WIRELESSLY INTERMEDIATING AN INTRAORAL SCANNER AND A DATA PROCESSING DEVICE**
ELEKTRONISCHE VORRICHTUNG UND VERFAHREN ZUR DRAHTLOSEN VERMITTLUNG EINES INTRAORALEN SCANNERS UND EINER DATENVERARBEITUNGSVORRICHTUNG
DISPOSITIF ÉLECTRONIQUE ET PROCÉDÉ POUR L'INTERMÉDIATION SANS FIL D'UN SCANNER INTRA-BUCCAL ET D'UN DISPOSITIF DE TRAITEMENT DE DONNÉES

(30) Priority: 18.02.2022 KR 20220021723
(43) Date of publication of application: 25.12.2024
(73) Proprietor: Medit Corp., Seoul 07207 (KR)
(72) Inventor: KIM, Hyeongseok, Seoul 07207 (KR); YOON, Sung Mun, Seoul 07207 (KR)
(74) Representative: Kim Kang, Jae Hee
(86) International application number: PCT/KR2023/002153
(87) International publication number: WO 2023/158192

(56) References cited:
- CN-B- 107 231 642
- JP-A- 2015 223 364
- KR-A- 20170 111 848
- KR-A- 20190 141 475
- KR-A- 20190 141 475
- KR-B1- 101 176 770
- KR-B1- 101 974 719
- US-A1- 2021 127 979
- HOLT KRIS: "TP-Link's new WiFi 6E router has motorized antennas that follow your devices", 4 January 2022 (2022-01-04), XP093256207, Retrieved from the Internet <URL:https://www.engadget.com/tp-link-router-antennas-automatically-move-wifi-6e-151032290.html> [retrieved on 20250305]
- TP-LINK: "CES 2022 | TP-Link Introduces Archer AXE300 - The Fastest WiFi 6E Router to Date", 4 January 2022 (2022-01-04), XP093256215, Retrieved from the Internet <URL:https://www.tp-link.com/uk/press/news/19849/> [retrieved on 20250305]

## Description

### Technical Field

The disclosure relates to an electronic device and an operation method of the electronic device. More particularly, the disclosure relates to an electronic device mediating between a wireless scanner and a data processing device that processes scan data obtained by the wireless scanner and an operation method of the electronic device.

### Background Art

There are various fields of dental treatment for patients. For dental treatment of a patient, it is important to accurately identify the state of the patient's oral cavity. In order to accurately identify the state of a patient's oral cavity, dental computer-aided design (CAD)/computer-aided manufacturing (CAM) technology is widely used. Specifically, the most important thing in dental treatment using CAD/CAM is to obtain precise three-dimensional (3D) data about the shape of the interior or exterior of an oral cavity, such as a patient's teeth, gums, or jawbone.

Wireless scanners may be used to obtain 3D data about an oral cavity. 3D optical scanners are widely used as wireless scanners. 3D optical scanners may obtain images of an oral cavity by using light reflected from an object. Images may be transmitted to a data processing device to process images obtained from wireless scanners.

Recently, there has been a trend in the dental industry to increase user convenience by connecting a wireless scanner that scans patients' oral cavity and a data processing device wirelessly rather than through wires. An electronic device mediating data and/or control signals between a wireless scanner and a data processing device may be required to wirelessly connect the wireless scanner to the data processing device.

KR20190141475A discloses a dental uncompressed image and data transmission system for transmitting an image taken by an oral camera.

Holt, Kris: "TP-Link's new WiFi 6E router 2-5,12 A61C19/04 has motorized antennas that follow your devices" 4 January 2022.

### Disclosure

### Technical Problem

The disclosure provides an electronic device mediating between a wireless scanner and a data processing device that processes scanning signals obtained by the wireless scanner to facilitate communication between the wireless scanner and the data processing device, and an operation method of the electronic device.

### Technical Solution

To solve the technical problem, there is provided an electronic device according to claim 1 and an operation method of an electronic device according to claim 10.

According to an embodiment, a housing of the electronic device may include a hinge for controlling an inclination of the antenna.

According to an embodiment, the antenna may be disposed to have an inclination on a side of a housing of the electronic device, and the side of the housing of the electronic device may be installed to have an inclination corresponding to an inclination of the antenna.

According to an embodiment, the wireless communication module may include a second communication module operating in a 60 GHz signal transmission/reception band, and the processor may execute the one or more instructions to obtain at least one of the inclination direction of the antenna or the inclination direction of the housing of the electronic device where the antenna is installed for which a strength of signal received from the second communication module exceeds a threshold, and mechanically control at least one of the antenna or the housing of the electronic device according to at least one of the obtained inclination direction of the antenna or the obtained inclination direction of the housing.

According to an embodiment, the processor may execute the one or more instructions to detect a communication state with the wireless scanner, and provide user feedback guiding to adjust a location or a direction of at least one of the wireless scanner or the electronic device in response to the detected communication state.

According to an embodiment, the processor may execute the one or more instructions to provide the user feedback guiding to adjust the location or the direction of at least one of the wireless scanner or the electronic device when the detected communication state indicates that communication is possible in a 2.4 GHz range and that communication is impossible in a 60 GHz range.

According to an embodiment, the processor may execute the one or more instructions to detect an operating state of the wireless scanner, and control cooling means of the electronic device according to the detected operating state of the wireless scanner.

According to an embodiment, the processor may execute the one or more instructions to control cooling means of the electronic device to operate when an operating state of the wireless scanner indicates that the wireless scanner is operating, and control the cooling means of the electronic device not to operate when the operating state of the wireless scanner indicates that the wireless scanner is waiting to operate.

According to an embodiment, the processor may execute the one or more instructions to detect the operating state of the wireless scanner by receiving information about the operating state of the wireless scanner from the wireless scanner.

According to an embodiment, a scam system includes a wireless scanner configured to scan an object, and including a first wireless communication module configured to communicate to transmit scan data to an electronic device, wherein the first wireless communication module is disposed on a rear surface of the wireless scanner for communication with the second wireless communication module of the electronic device, and an electronic device including a second wireless communication module configured to communicate to receive the scan data from the wireless scanner, wherein the second wireless communication module is disposed to operate at a specified angle range with the rear surface of the wireless scanner so as to communicate with the first wireless communication module.

According to an embodiment, an operation method of an electronic device mediating between a wireless scanner and a data processing device processing scan data obtained by the wireless scanner includes detecting a communication state with the wireless scanner through a wireless communication module of the electronic device, and mechanically controlling at least one of an inclination direction of an antenna, an inclination direction of a housing of the electronic device where the antenna is installed, or a location of the electronic device according to the detected communication state.

According to an embodiment, an electronic device mediating between a wireless scanner and a data processing device processing a scan image obtained by the wireless scanner includes an upper case, a lower case, a wireless communication module for transmission/reception of the scan image to and from the wireless scanner, and a main printed circuit board (PCB) processing the scan image received from the wireless scanner and controlling the processed scan image to be transmitted to the data processing device, and an antenna of the wireless communication module is tilted to have a certain angle to enable communication with a communication module of the wireless scanner that transmits the scan image.

According to an embodiment, a sidewall of the upper case of the electronic device adjacent to the antenna of the wireless communication module may be inclined by a certain angle formed by the antenna.

The data processing device may further include an antenna driving controller connected to the antenna to control an orientation of the antenna of the wireless communication module according to a communication state of the wireless communication module.

The antenna driving controller may control at least one of rotation or tilt of the antenna.

The lower case of the electronic device may have a hinge on a bottom surface to adjust an angle, to control an inclination of the antenna of the wireless communication module.

The lower case of the electronic device may further include a rotating plate on the bottom surface to rotate the lower case, to control a direction of the antenna of the wireless communication module.

According to an embodiment, the antenna of the wireless communication module may be disposed to have an angle inclination of 10 degrees to 30 degrees.

According to an embodiment, a heat sink may be disposed in the wireless communication module to dissipate heat generated according to an operation of the wireless communication module.

According to an embodiment, the wireless communication module and the main PCB may be in contact with a metal frame to dissipate heat.

According to an embodiment, an air discharge hole is provided in the upper case, cooling means is disposed below the air discharge hole, and heat generated from the wireless communication module and the main PCB may be discharged to the outside of the electronic device through the cooling means and the air discharge hole.

According to an embodiment, the electronic device may include a support accommodated between the upper case and the lower case, the wireless communication module may be installed on one side of the support, a structure having a height may be installed on the other side of the support, a sub-PCB may be installed on the structure, and the support may include a hole for passing heat from the main PCB located below the support.

According to an embodiment, one or more ventilation holes may be provided in the lower case to ventilate external air.

### Advantageous Effects

According to embodiments, an electronic device mediating between a wireless scanner that scans an object and a data processing device that processes scan data obtained by the wireless scanner may allow a user to operate the wireless scanner more conveniently by removing a line for connection from the wireless scanner to the data processing device.

According to embodiments, locations of communication modules for communication between the wireless scanner and the wireless hub may be appropriately disposed in the wireless scanner and the wireless hub, thereby facilitating signal transmission and reception between the wireless scanner and the wireless hub.

According to embodiments, user feedback may be provided when signal transmission and reception between the wireless scanner and the wireless hub are not smooth, thereby providing a guide to the user to facilitate signal transmission and reception between the wireless scanner and the wireless hub and adjust a location or a direction of the wireless scanner or the wireless hub.

According to embodiments, when signal transmission and reception between the wireless scanner and the wireless hub are not smooth, this may be detected and automatically adjusted to control an antenna inclination or direction of the wireless hub, thereby conveniently facilitating signal transmission and reception between the wireless scanner and the wireless hub without user intervention.

According to embodiments, the wireless hub with a structure capable of effectively dissipating heat generated from circuit components within the wireless hub may increase the operation efficiency of the wireless hub.

### Description of Drawings

The present disclosure may be readily understood with a combination of the following detailed descriptions and the accompanying drawings, wherein reference numbers refer to structural elements.
FIG. 1 is a diagram for explaining a digital oral model processing system according to an embodiment.
FIG. 2 is a schematic block diagram of each device in a system including a wireless scanner, a wireless hub, and a data processing device according to various embodiments.
FIG. 3 is a detailed block diagram of a wireless scanner and a wireless hub according to an embodiment.
FIG. 4 illustrates an example in which a system including a wireless scanner, a wireless hub, and a data processing device is arranged in an actual use environment.
FIG. 5 is a reference diagram for explaining an appropriate location of a second communication module disposed in a wireless scanner according to an embodiment.
FIGS. 6A and 6B are reference diagrams for explaining an appropriate location of a second communication module disposed in a wireless hub according to an embodiment.
FIG. 7A illustrates an example in which a second communication module is disposed to be inclined at a certain angle in a wireless hub according to an embodiment.
FIG. 7B illustrates another example in which a second communication module is disposed to be inclined at a certain angle in a wireless hub according to an embodiment.
FIG. 8 illustrates another example in which a second communication module is disposed to be inclined at a certain angle in a wireless hub according to an embodiment.
FIG. 9 is a flowchart illustrating an operation of providing a guide to a user by transmitting state information of a wireless hub to a data processing device according to an embodiment.
FIG. 10 illustrates an example of a wireless hub that enables automatic adjustment of a radiation region of a second communication module according to communication state information of the wireless hub according to an embodiment.
FIG. 11 is a reference diagram for explaining an operation of a wireless hub that enables automatic adjustment of rotation or inclination of an antenna of a second communication module according to communication state information of the wireless hub according to an embodiment.
FIG. 12 is a reference diagram for explaining an operation of a wireless hub that enables automatic adjustment of a hinge installed in the wireless hub according to communication state information of the wireless hub according to an embodiment.
FIG. 13 is a reference diagram for explaining an operation of a wireless hub that enables automatic adjustment of a rotating plate installed in the wireless hub according to communication state information of the wireless hub according to an embodiment.
FIG. 14 is a reference diagram for explaining a signal transmission/reception range according to a location relationship between a wireless scanner and a wireless hub according to various examples.
FIG. 15 is a flowchart illustrating an operation of a method of providing user feedback according to a communication state between a wireless scanner and a wireless hub according to an embodiment.
FIG. 16A is a reference diagram for explaining a notification operation of a data processing device in a state in which both a first communication module and a second communication module are not communicable because a wireless scanner is out of a 2.4 GHz band range according to an embodiment.
FIG. 16B is a reference diagram for explaining a notification operation of a data processing device in a state in which a wireless scanner is in a 2.4 GHz band range but is out of a 60 GHz band range, that is, a first communication module is communicable but a second communication module is not communicable.
FIG. 16C is a reference diagram for explaining a notification operation of a data processing device in a state in which a wireless scanner enters a 60 GHz band range and is communicable with both a first communication module and a second communication module according to an embodiment.
FIG. 17 is a flowchart illustrating an operation of a method of controlling heat generation in a wireless hub according to an embodiment.
FIG. 18 is a reference diagram for describing scanner mode information according to an embodiment.
FIG. 19 is a flowchart illustrating an example of an operation method of a wireless hub according to a noise measurement value according to an embodiment.
FIG. 20 is a flowchart illustrating an operation of a method of displaying state information of a wireless scanner according to an embodiment.
FIG. 21A illustrates an example of a structure of a wireless hub for eliminating heat generation of components according to an embodiment.
FIG. 21B illustrates an example of an exploded view of a wireless hub according to an embodiment.
FIG. 22 is a reference diagram for describing a structure of a wireless hub for eliminating heat generation of components according to an embodiment.
FIG. 23 is a reference diagram for describing a connection structure for a wireless hub to communicate with a data processing device according to an embodiment.

### Best Mode

### Mode for Invention

The present specification describes the principles of the present application and discloses embodiments to allow one of ordinary skill in the art to practice the present application. The embodiments may be implemented in various forms.

Throughout the specification, like reference numerals denote like elements. The present specification does not describe all elements of the embodiments, and generic content in the technical field of the present application or redundant content of the embodiments is omitted. The term "part" or "portion" used in the specification may be implemented by software or hardware, and according to embodiments, a plurality of "parts" or "portions" may be implemented as one unit or element, or alternatively, one "part" or "portion" may include a plurality of units or elements. Hereinafter, operation principles and embodiments of the present application will be described with reference to accompanying drawings.

In the present specification, an image may include an image (hereinafter, referred to as an "intraoral image") indicating at least one tooth, an oral cavity including at least one tooth, or an oral cavity including gingiva.

Also, in the present specification, an image may include a 2-dimensional (2D) image of an object or a 3D model or a 3D image stereoscopically indicating an object. In addition, in the present specification, an image may refer to data required to express an object in two dimensions or three dimensions, for example, raw data obtained by at least one image sensor. Specifically, the raw data is data obtained to generate an intraoral image, and may be data (e.g., 2D data) obtained by at least one image sensor included in the intraoral scanner when scanning the oral cavity of a patient, which is an object, by using a wireless scanner.

The 'object' herein may include a tooth, gingiva, at least a partial region of an oral cavity, and/or an artificial structure insertable into an oral cavity (e.g., an orthodontic device, an implant, an artificial tooth, an orthodontic auxiliary tool inserted into the oral cavity, etc.) Here, the orthodontic device may include at least one of a bracket, an attachment, an orthodontic screw, a lingual orthodontic device, or a removable orthodontic retainer.

Hereinafter, embodiments will be described in detail with reference to the drawings.

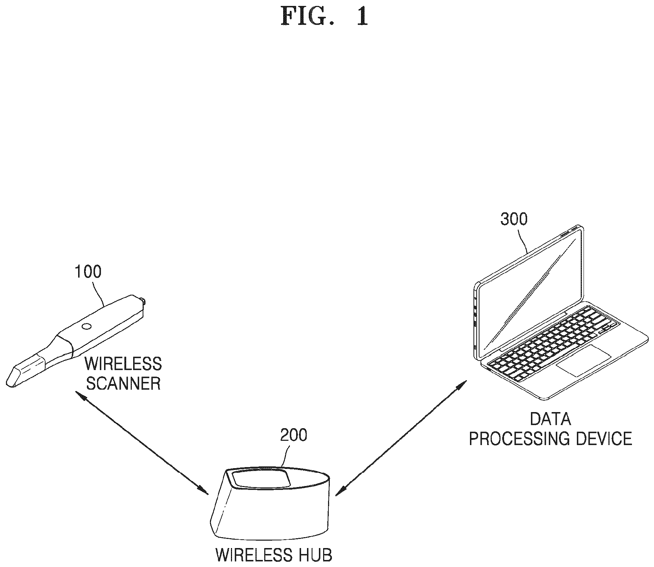

FIG. 1 is a diagram for explaining a digital oral model processing system according to an embodiment.

Referring to FIG. 1, the digital oral model processing system may include a wireless scanner 100, a wireless hub 200, and a data processing device 300.

The wireless scanner 100 is a device that scans an object, and the object may include any object or body that is a target of scanning. For example, the object may include at least a part of the patient's body, including the mouth or face, or a model of teeth. The wireless scanner 100 may include a handheld scanner that scans the object while a user holds the handheld scanner in his/her hand.

For example, the wireless scanner 100 that is a kind of handheld scanner may be a device for obtaining an image of an oral cavity including at least one tooth, by being inserted into the oral cavity and scanning the tooth in a contactless manner. In addition, the wireless scanner 100 may have a shape capable of being drawn in and out of an oral cavity, and scans the oral cavity of a patient by using at least one camera (e.g., an optical camera, etc.) The wireless scanner 100 may obtain, as raw data, surface information about an object, in order to image the surface of at least one of teeth, gingiva, an artificial structure (e.g., an orthodontic device including a bracket and a wire, an implant, an artificial tooth, an orthodontic auxiliary tool inserted into the oral cavity, etc.) The wireless scanner 100 may have the shape capable of easily being drawn in and out of the oral cavity to be suitable for scanning the oral cavity, but may be capable of scanning a body part such as the patient's face.

The wireless scanner 100 may obtain image data by using an optical triangulation method, a confocal method, or other methods.

The image data obtained by the wireless scanner 100 may be transmitted to the data processing device 300 for processing, analyzing, and displaying a 3D oral model. In this regard, the image data obtained by the wireless scanner 100 may be transmitted to the data processing device 300 through a wired communication network. However, when the wireless scanner 100 and the data processing device 300 are connected by wired, a cable connected to the wireless scanner 100 may limit an operation of the user and cause inconvenience when the user grips and operates the wireless scanner 100. Accordingly, according to various embodiments of the disclosure, a wireless hub 200 capable of connecting the wireless scanner 100 to the data processing device 300 through the wireless communication network is provided.

The wireless hub 200 may mediate communication between the wireless scanner 100 and the data processing device 300 by communicating with the wireless scanner 100 through wireless communication and communicating with the data processing device 300 through wired or wireless communication. Specifically, the wireless hub 200 may transmit a scanning signal received from the wireless scanner 100 through wireless communication to the data processing device 300 through wired or wireless communication. The term "wireless hub" is a term used to distinguish it from the data processing device 300, and the wireless hub 200 may be any electronic device mediating between the wireless scanner 100 and the data processing device 300. For example, the wireless hub 200 may be referred to as an electronic device, a mediating device, a communication device, or a computing device to mediate between the wireless scanner 100 and the data processing device 300.

The wireless hub 200 is required to effectively receive a wireless signal transmitted from the wireless scanner 100 through wireless communication. However, constraints in an environment where a wireless scanner and a wireless hub are actually arranged, for example, in a dental clinic space, may require structures and arrangements that may effectively enable communication of wireless signals between the wireless scanner 100 and the wireless hub 200.

According to an embodiment, the wireless hub 200 may include at least one antenna arranged or controlled to have at least one of a location or a direction suitable for receiving scan data.

According to an embodiment, the wireless hub 200 may have a hinge attached to a housing of the wireless hub 200 to control an inclination of an antenna.

According to an embodiment, the antenna of the wireless hub 200 may be disposed to have the inclination on the housing side of the wireless hub 200, and the housing side of the wireless hub 200 may be installed to have an inclination corresponding to the inclination of the antenna.

According to an embodiment, the wireless hub 200 may detect a communication state with the wireless scanner 100 and mechanically control at least one of an inclination direction of the antenna, an inclination direction of the housing of the wireless hub 200 in which the antenna is installed, or a location of the wireless hub 200 according to the detected communication state.

According to an embodiment, the wireless hub 200 may obtain at least one of the inclination direction of the antenna or the inclination direction of the housing of the wireless hub 200 in which the antenna is installed for which a strength of a signal received from the communication module exceeds a threshold, and mechanically control at least one of the antenna or the housing of the wireless hub 200 according to the obtained at least one of the inclination direction of the antenna or the obtained inclination direction of the housing.

According to an embodiment, the wireless hub 200 may detect the communication state with the wireless scanner 100 and provide user feedback guiding to adjust a location or a direction of at least one of the wireless scanner 100 or the electronic device in response to the detected communication state.

According to an embodiment, the wireless hub 200 may provide user feedback guiding to adjust a location or a direction of at least one of the wireless scanner 100 or the electronic device as the detected communication state indicates that communication is possible in a 2.4 GHz range and communication is impossible in a 60 GHz range.

According to an embodiment, the wireless hub 200 may detect an operating state of wireless scanner 100 and control cooling means of the electronic device according to the detected operating state of the wireless scanner 100.

According to an embodiment, the wireless hub 200 may control cooling means of the wireless hub 200 to operate when the operating state of the wireless scanner 100 indicates that the operating state of the wireless scanner 100 is in operation, and control the cooling means of the wireless hub 200 to not operate when the operating state of the wireless scanner 100 indicates that the operating state of the wireless scanner 100 is waiting to operate.

According to an embodiment, the wireless hub 200 may detect the operating state of the wireless scanner 100 by receiving information about the operating state of the wireless scanner 100 from the wireless scanner 100.

The wireless hub 200 may transmit the image data received from the wireless scanner 100 to the data processing device 300 through wired or wireless communication.

According to an embodiment, the wireless hub 200 may be connected to the data processing device 300 by wired to receive power supply from the data processing device 300 connected by wired.

The data processing device 300 may be any electronic device connected to the wireless hub 200 through a wired or wireless communication network, receiving a two-dimensional (2D) image obtained by scanning the oral cavity from the wireless hub 200, and generating, processing, displaying, and/or transmitting an intraoral image based on the received 2D image.

The data processing device 300 may generate at least one of information generated by processing 2D image data or the intraoral image generated by processing the 2D image data based on the 2D image data received from the wireless hub 200, and display the generated information and intraoral image through a display.

The data electronic device 300 may be a computing device such as a smart phone, a laptop computer, a desktop computer, a personal digital assistant (PDA), or a tablet personal computer (PC), but is not limited thereto. In addition, the data electronic device 300 may be in the form of a server, etc. for processing an intraoral image.

In addition, the wireless scanner 100 may transmit raw data obtained through scanning to the data processing device 300 through the wireless hub 200 as it is. In this case, the data processing device 300 may generate a 3D intraoral image representing the oral cavity in three dimensions based on the received raw data. In addition, the '3D intraoral image' may be generated by modeling an internal structure of the oral cavity in three dimensions based on the received raw data, and thus, the '3D intraoral image' may be referred to as a '3D intraoral model', or a' digital intraoral model'. Hereinafter, a model or an image representing the oral cavity in two dimensions or three dimensions is collectively referred to as an 'intraoral image'.

In addition, the data processing device 300 may analyze, process, display, and/or transmit the generated intraoral image to an external device.

According to an embodiment, the data processing device 300 may receive information about the operating state of the wireless scanner 100 from the wireless hub 200 and display the information about the operating state of the wireless scanner 100.

According to an embodiment, the data processing device 300 may receive communication state information between the wireless hub 200 and the wireless scanner 100 from the wireless hub 200, and display the received communication state information, thereby providing the user with a guide indicating that the location or the direction of the wireless hub 200 or the wireless scanner 100 needs to be adjusted for smooth signal transmission and reception between the wireless hub 200 and the wireless scanner 100.

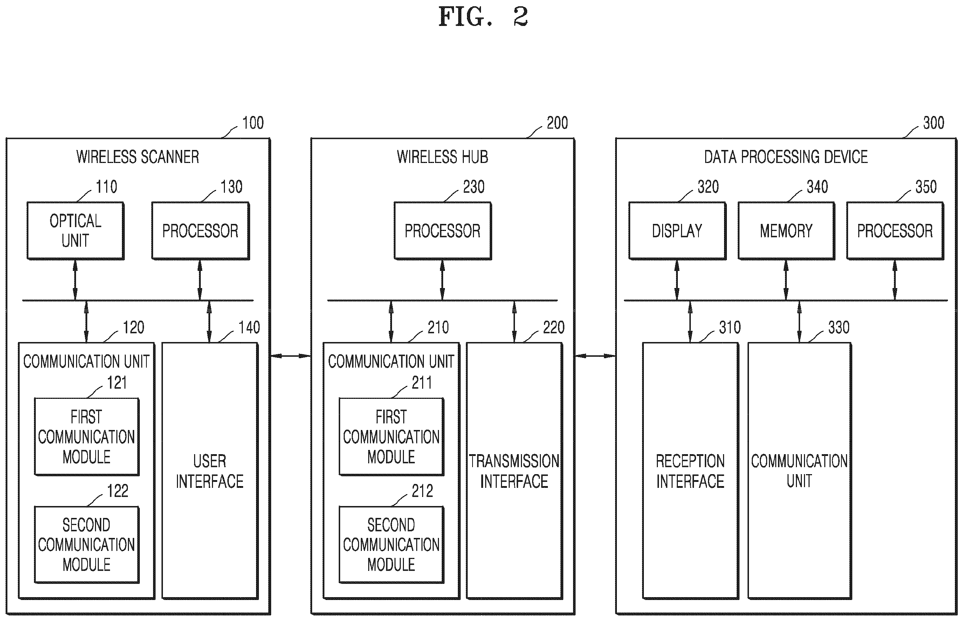

FIG. 2 is a schematic block diagram of each device in a system including the wireless scanner 100, the wireless hub 200, and the data processing device 300 according to various embodiments.

Referring to FIG. 2, the system may include the wireless scanner 100, the wireless hub 200, and the data processing device 300.

The wireless scanner 100 may include an optical unit 110, a communication unit 120, a processor 130, and a user interface 140.

The optical unit 110 may include at least one camera, obtain at least one image by photographing an object, and generate image data to be transmitted to the wireless hub 200.

The communication unit 120 may perform wireless communication with the wireless hub 200 through a plurality of communication channels. Here, the communication channel may refer to a communication network transmitting and receiving wireless signals through a certain frequency band. Specifically, the communication channel may be a communication network transmitting and receiving wireless signals of a frequency band defined according to a certain wireless communication standard. Here, the wireless communication standard may be a communication standard such as Wireless Gigabit (WiGig), Bluetooth, Wi-Fi, Bluetooth Low Energy (BLE), near field communication (NFC)/radio frequency identification (RFID), Wi-Fi direct (WFD), ultra-wideband (UWB), or ZigBee. In addition, a communication network of a certain frequency band or conforming to a certain communication standard may be referred to as a communication channel. In addition, the communication unit 120 may also perform wired communication with the wireless hub 200. However, in the embodiment, an example in which the communication unit 120 performs wireless communication with the wireless hub 200 will be illustrated and described.

The communication unit 120 may include a first communication module 121 performing wireless communication in a first frequency band and a second communication module 122 performing wireless communication in a second frequency band. The first frequency band may be a frequency band defined according to communication standards such as Bluetooth, Wi-Fi, BLE, NFC/RFID, Wi-Fi Direct, UWB, or ZIGBEE. The second frequency band may be a frequency band different from the first frequency band.

The first communication module 121 may perform bi-directional wireless communication. Specifically, the first communication module 121 may be responsible for transmission and reception of a control signal through a first communication network having the first frequency band. The control signal may be a signal including settings, information, requests, and/or commands necessary for the wireless scanner 100 to obtain an image of an oral cavity. As another example, the control signal may be a signal including settings, information, requests, and/or commands necessary for the wireless scanner 100 to transmit the obtained image to the wireless hub 200.

According to an embodiment, the first communication module 121 may transmit image data temporarily or for a certain period of time when a communication state of the second communication module 122 is not smooth. The image data transmitted through the first communication module 121 may include a 3D intraoral image obtained by processing raw data obtained by scanning an object by the wireless scanner 100.

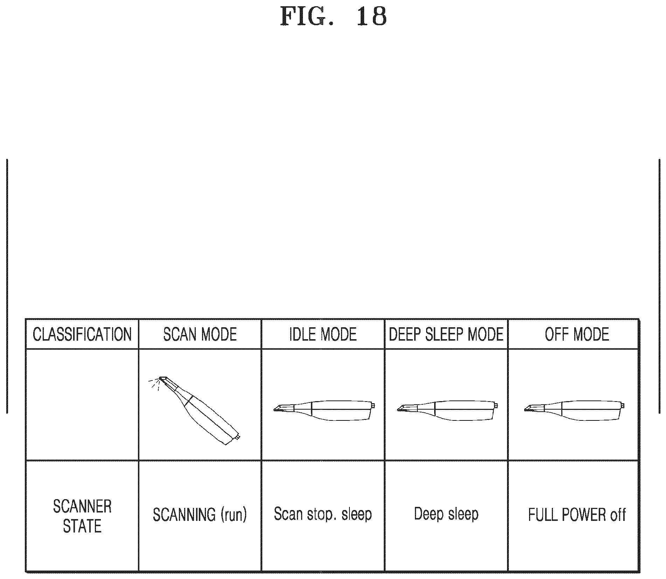

According to an embodiment, the first communication module 121 may transmit information about a scanner mode of the wireless scanner 100 to the wireless hub 200 as a control signal. The information about the scanner mode may include at least one of a scan mode, an idle mode, a deep sleep mode, or an off mode, which will be described in detail with reference to FIG. 18.

The first communication module 121 may transmit the control signal to the wireless hub 200 through a wireless communication network of the first frequency band, for example, a 2.4 GHz band. As another example, the first communication module 121 may transmit the control signal to the wireless hub 200 through a wireless communication network of the first frequency band, for example, a 5 GHz band. As another example, the first communication module 121 may transmit the control signal to the wireless hub 200 through a wireless communication network having an Industrial Scientific and Medical (ISM) frequency band, for example, a 4000 MHz frequency band, or a 900 MHz frequency band.

In addition, the first communication module 121 may receive the control signal from the wireless hub 200.

The second communication module 122 may perform uni-directional wireless communication or bi-directional wireless communication.

Specifically, when fast transmission of image data is desired, the second communication module 122 may perform uni-directional wireless communication. For example, the second communication module 122 may perform bi-directional wireless communication from the wireless scanner 100 to the wireless hub 200 in a 60 GHz frequency band for fast transmission of the image data.

In addition, the second communication module 122 may transmit and receive wireless signals in the form of a millimeter wave. Here, the millimeter wave refers to a wireless signal having a wavelength of 1 millimeter to 10 millimeters. Accordingly, the second communication module 122 that transmits and receives millimeter waves may transmit and receive wireless signals in a bandwidth of 30 GHz to 300 GHz. Specifically, the second communication module 122 may rapidly transmit a large amount of image data by transmitting and receiving millimeter-wave wireless signals.

Specifically, the second communication module 122 may transmit and receive wireless signals of a frequency band according to WiGig. Specifically, the second communication module 122 may transmit and receive wireless signals in a frequency band of 802.11ad of WiGig. Here, wireless communication through the Institute of Electrical and Electronics Engineers (IEEE) 802.11ad frequency band may be referred to as WiGig. In addition, WiGig may support a data transmission rate of up to 7 giga bits per second (Gbps). In addition, WiGig may stably perform wireless communication within a distance of 10 m or less when maximally supporting the strength of a wireless signal transmitted through beamforming. In addition, WiGig may support transmission and reception of data encrypted by using an encryption algorithm, and in this case, may provide an improved security function. Specifically, WiGig may provide an improved security function by using Galois/Counter Mode of the Advanced Encryption Standard (AES) encryption algorithm. For example, the second communication module 122 may transmit data in a 60-GHz band by using a wireless communication network conforming to WiGig.

As described above, the second communication module 122 is used to transmit a large amount of image data at high speed, but the image data may be transmitted through the first communication module 121 when the communication state of the second communication module 122 is not smooth. The image data transmitted through the first communication module 121 may include a 3D intraoral image obtained by processing raw data obtained by scanning an object by the wireless scanner 100. Also, the transmission of image data through the first communication module 121 may be temporarily performed. Therefore, when the communication state of the second communication module 122 is temporarily transmitted through the first communication module 121, the image data may be transmitted again through the second communication module 122 when the communication state of the second communication module 122 is smooth.

The processor 130 may perform an intended operation by executing at least one instruction. Specifically, the processor 130 may control an operation of photographing (or scanning) an oral cavity, an operation of obtaining an image of the oral cavity, and/or an operation of transmitting data corresponding to the obtained image. In addition, when it is described that the processor 130 performs a certain operation, this may mean that a processor 130 directly performs the above-described operations by executing at least one instruction, as well as that the processor 130 controls other components such that the above-described operations are performed.

Specifically, the processor 130 may include random-access memory (RAM) (not shown), which stores signals or data input from a source external to the wireless scanner 100 or is used as a storage for various operations performed by the wireless scanner 100, read-only memory (ROM) (not shown) storing a control program and/or a plurality of instructions for controlling the wireless scanner 100, and at least one processor (hereinafter, referred to as an 'internal processor') (not shown) configured to execute at least one instruction. Specifically, the processor 130 may be implemented to include at least one internal processor and a memory device (e.g., RAM, ROM, etc.) for storing at least one of programs, instructions, signals, or data to be processed or used by the internal processor.

Also, the processor 130 may include a graphics processing unit (GPU) (not shown) for graphics processing on a video. In addition, the processor 130 may be implemented as a system-on-chip (SoC) in which a core (not shown) and a GPU (not shown) are integrated. In addition, the processor 130 may include a single processor core (single-core) or a plurality of processor cores (multi-core). In addition, the processor 130 may include a field-programmable gate array (FPGA), which is a semiconductor device including a designable logic device and a programmable internal circuit, and may perform high-speed image processing by using the FPGA.

The processor 130 obtains image data corresponding to the at least one image obtained by the optical unit 110, controls a control signal related to at least one of obtaining and transmission operations of the at least one image to be transmitted to and from the wireless hub 200 through the first communication module 121 performing wireless communication in the first frequency band, and controls the image data to be transmitted to the wireless hub 200 through the second communication module 122 performing wireless communication in the second frequency band.

In addition, the wireless scanner 100 may further include a user interface 140 capable of receiving a user input.

For example, the user interface 140 may include an input device including keys corresponding to certain operations or requests. For example, the input device included in the user interface 140 may be formed as at least one button, a touch sensor, a touch pad, etc. Alternatively, the user interface 140 may include a speech recognition sensor, and may receive a user voice, and recognize a user input corresponding to a certain operation or request based on the received user voice.

The wireless hub 200 may include a communication unit 210, a transmission interface 220, and a processor 230.

The communication unit 210 may perform wireless communication with the wireless scanner 100 through a plurality of communication channels. Here, the communication channel may refer to a communication network for transmitting and receiving wireless signals through a certain frequency band. Specifically, the communication channel may be a communication network for transmitting and receiving wireless signals of a frequency band defined according to a certain wireless communication standard. Here, the wireless communication standard may be a communication standard such as Wireless Gigabit (WiGig), Bluetooth, Wi-Fi, BLE, NFC/RFID, Wi-Fi Direct, UWB, or ZIGBEE. In addition, a communication network of a certain frequency band or conforming to a certain communication standard may be referred to as a communication channel.

The communication unit 210 may include a first communication module 211 performing wireless communication in a first frequency band and a second communication module 212 performing wireless communication in a second frequency band. For example, the first frequency band may be a frequency band defined according to communication standards such as Bluetooth, Wi-Fi, BLE, NFC/RFID, Wi-Fi Direct, UWB, or ZIGBEE. The second frequency band may be a frequency band different from the first frequency band.

The first communication module 211 may perform bi-directional wireless communication. Specifically, the first communication module 211 may be responsible for transmission and reception of a control signal through a first communication network having the first frequency band. The control signal may be a signal including settings, information, requests, and/or commands necessary for the wireless scanner 100 to obtain an image of an oral cavity. As another example, the control signal may be a signal including settings, information, requests, and/or commands necessary for the wireless scanner 100 to transmit the obtained image to the wireless hub 200.

According to an embodiment, the first communication module 211 may transmit image data temporarily or for a certain period of time when a communication state of the second communication module 212 is not smooth. The image data transmitted through the first communication module 211 may include a 3D intraoral image obtained by processing raw data obtained by scanning an object by the wireless scanner 100.

According to an embodiment, the first communication module 211 may receive information about a scanner mode of the wireless scanner 100 from the wireless scanner 100 as a control signal. The information about the scanner mode may include at least one of a scan mode, an idle mode, a deep sleep mode, or an off mode.

The first communication module 211 may transmit the control signal to the wireless scanner 100 through a wireless communication network of the first frequency band, for example, a 2.4 GHz band. As another example, the first communication module 121 may transmit the control signal to the wireless scanner 100 through a wireless communication network of the first frequency band, for example, a 5 GHz band. As another example, the first communication module 211 may transmit the control signal to the wireless scanner 100 through a wireless communication network having an ISM frequency band, for example, a 4000 MHz frequency band, or a 900 MHz frequency band.

In addition, the first communication module 211 may receive the control signal from the wireless scanner 100.

The second communication module 212 may perform uni-directional wireless communication or bi-directional wireless communication.

Specifically, when fast transmission of image data is desired, the second communication module 212 may perform uni-directional wireless communication. For example, the second communication module 212 may perform bi-directional wireless communication from the wireless scanner 100 to the wireless hub 200 in a 60 GHz frequency band for fast transmission of the image data.

In addition, the second communication module 212 may transmit and receive wireless signals in the form of a millimeter wave. Here, the millimeter wave refers to a wireless signal having a wavelength of 1 millimeter to 10 millimeters. Accordingly, the second communication module 212 that transmits and receives millimeter waves may transmit and receive wireless signals in a bandwidth of 30 GHz to 300 GHz. Specifically, the second communication module 212 may rapidly transmit a large amount of image data by transmitting and receiving millimeter-wave wireless signals.

Specifically, the second communication module 212 may transmit and receive wireless signals of a frequency band according to WiGig. Specifically, the second communication module 212 may transmit and receive wireless signals in a frequency band of 802.11ad of WiGig. Here, wireless communication through the IEEE 802.11ad frequency band may be referred to as WiGig. In addition, WiGig may support a data transmission rate of up to 7 Gbps. In addition, WiGig may stably perform wireless communication within a distance of 10 m or less when maximally supporting the strength of a wireless signal transmitted through beamforming. In addition, WiGig may support transmission and reception of data encrypted by using an encryption algorithm, and in this case, may provide an improved security function. Specifically, WiGig may provide an improved security function by using Galois/Counter Mode of the AES encryption algorithm. For example, the second communication module 212 may transmit data in a 60-GHz band by using a wireless communication network conforming to WiGig.

As described above, the second communication module 212 is used to transmit a large amount of image data at high speed, but the image data may be transmitted through the first communication module 211 when the communication state of the second communication module 212 is not smooth. The image data transmitted through the first communication module 211 may include a 3D intraoral image obtained by processing raw data obtained by scanning an object by the wireless scanner 100. Also, the transmission of image data through the first communication module 211 may be temporarily performed. Therefore, when the communication state of the second communication module 212 is temporarily transmitted through the first communication module 211, the image data may be transmitted again through the second communication module 212 when the communication state of the second communication module 212 is smooth.

The transmission interface 220 may transmit data to the data processing device 300 through wired or wireless communication. The transmission interface 220 may include at least one port for connecting to the data processing device 300 through a wired cable in order to communicate with the data processing device 300 by wired. Accordingly, the transmission interface 220 may communicate with the data processing device 300 by wired connected through the at least one port. When the transmission interface 220 is connected to the data processing device 300 through the wired cable, the transmission interface 220 may receive power from the data processing device 300 through the wired cable.

The processor 230 may perform an intended operation by performing at least one instruction. Specifically, the processor 230 may control an operation of receiving scan data from the wireless scanner 100 and an operation of transmitting the scan data to the data processing device 300, an operation of receiving the control signals or the information about the operating state of the wireless scanner 100 from the wireless scanner 100, and an operation of transmitting the control signals or the information about the operating state of the wireless scanner 100 to the data processing device 300. In addition, when it is described that the processor 230 performs a certain operation, this may mean that the processor 230 directly performs the above-described operations by executing at least one instruction, as well as that the processor 130 controls other components such that the above-described operations are performed.

Specifically, the processor 230 may include RAM (not shown), which stores signals or data input from a source external to the wireless hub 200 or is used as a storage for various operations performed by the wireless hub 200, ROM (not shown) storing a control program and/or a plurality of instructions for controlling the wireless hub 200, and at least one processor (hereinafter, referred to as an 'internal processor') (not shown) configured to execute at least one instruction. Specifically, the processor 230 may be implemented to include at least one internal processor and a memory device (e.g., RAM, ROM, etc.) for storing at least one of programs, instructions, signals, or data to be processed or used by the internal processor.

Also, the processor 230 may include a GPU (not shown) for graphics processing on a video. In addition, the processor 230 may be implemented as an SoC in which a core (not shown) and a GPU (not shown) are integrated. In addition, the processor 230 may include a single processor core (single-core) or a plurality of processor cores (multi-core). In addition, the processor 230 may include a FPGA, which is a semiconductor device including a designable logic device and a programmable internal circuit, and may perform high-speed image processing by using the FPGA.

The processor 230 controls a control signal related to at least one of obtaining and transmission operations of the at least one image to be transmitted to and from the wireless scanner 100 through the first communication module 121 performing wireless communication in the first frequency band, and controls the image data to be transmitted to the wireless scanner 100 through the second communication module 122 performing wireless communication in the second frequency band.

In addition, the processor 230 may control the image data received from the wireless scanner 100 to be transmitted to the data processing device 300 through the transmission interface 220.

In addition, the processor 230 may control mode information of the wireless scanner 100 received from the wireless scanner 100 to be transmitted to the data processing device 300 through the transmission interface 220.

The data processing device 300 may include a reception interface 310, a display 320, a communication unit 330, a memory 340, and a processor 350.

The reception interface 310 may communicate with the transmission interface 220 of the wireless hub 200 to receive data from the wireless hub 200. The reception interface 310 may include at least one port for connecting to the wireless hub 200 through a wired cable in order to communicate with the wireless hub 200 by wired. Accordingly, the reception interface 310 may perform communication with the wireless hub 200 by wired connected through the at least one port.

The display 320 may display data processed by the data processing device 300. The display 320 displays a screen. Specifically, the display 320 may display a certain screen by the control of the processor 350. Specifically, the display 320 may display an intraoral image generated based on image data received through the wireless hub 200 from the wireless scanner 100. Alternatively, the display 320 may display a user interface screen including information related to the dental treatment of a patient.

The communication unit 330 may communicate with at least one external electronic device through a wired or wireless communication network. Specifically, the communication unit 330 may communicate with the wireless hub 200 by the control of the processor 350. The communication unit 330 may communicate with an external electronic device or a server connected through the wired or wireless communication network by the control of the processor 350.

The communication unit 330 may communicate with an external electronic device (e.g., the wireless scanner 100, a server, or an external medical device) through the wired or wireless communication network. The communication unit 330 may include at least one short-range communication module performing communication according to the communication standard, such as Bluetooth, Wi-Fi, BLE, NFC/RFID, Wi-Fi Direct, UWB, or ZIGBEE.

In addition, the communication unit 330 may further include a long-range communication module performing communication with a server for supporting long-range communication according to the long-range communication standard. Specifically, the communication unit 330 may include the long-range communication module performing communication through a network for Internet communication. For example, the communication unit 330 may include the long-range communication module performing communication through a communication network according to the communication standard, such as third generation (3G), fourth generation (4G), and/or fifth generation (5G).

The memory 340 may store at least one instruction. In addition, the memory 340 may store at least one instruction executed by the processor 350. In addition, the memory 340 may store at least one program executed by the processor 350. In addition, the memory 340 may store data (e.g., raw data obtained by scanning an oral cavity) received from the wireless scanner 100. Alternatively, the memory 340 may store an intraoral image representing the oral cavity in three dimensions. The memory 340 may receive the raw data obtained from the wireless scanner 10 and generate a 3D intraoral model based on the received data.

The processor 350 may perform the at least one instruction stored in the memory 340 to control an intended operation to be performed. Here, the at least one instruction may be stored in an internal memory included in the processor 350 or the memory 340 included in the data processing device 300 separately from the processor 350.

Specifically, the processor 350 may perform at least one instruction to control one or more components included in the data processing device 300 such that an intended operation is performed. Therefore, even though a case where the processor 350 performs certain operations is described as an example, it may mean that the processor 350 controls one or more components included in the data processing device 300 such that certain operations are performed.

Also, the processor 350 may include a GPU for graphics processing on a video. In addition, the processor 350 may be implemented as a SoC in which a core and a GPU are integrated. In addition, the processor 350 may include a single-core or a multi-core. For example, the processor 350 may be dual-core, triple-core, quad-core, hexa-core, octa-core, deca-core, dodeca-core, or hexadecimal-core.

In an embodiment, the processor 350 may generate an intraoral image based on the image data received from the wireless hub 200.

Specifically, by the control of the processor 350, the communication unit 330 may receive data obtained from the wireless scanner 100, for example, raw data obtained through oral cavity scanning, through the wireless hub 200. Then, the processor 350 may generate a 3D intraoral image representing the oral cavity in three dimensions, based on the received raw data. For example, the wireless scanner 100 may include at least one camera to restore a 3D image according to an optical triangulation method, an L camera corresponding to the left field of view and an R camera corresponding to the right field of view according to an embodiment. The wireless scanner 100 may obtain an L image data corresponding to the left field of view and R image data corresponding to the right field of view from the L camera and the R camera, respectively. Subsequently, the wireless scanner 100 may transmit raw data including the L image data and the R image data to the communication unit 330 of the data processing device 300.

In addition, the processor 350 may control the communication unit 330 to directly receive an intraoral image representing the oral cavity in three dimensions from an external server, a medical device, etc. In this case, the processor 350 may obtain a 3D intraoral image without generating the 3D intraoral image based on the raw data.

According to the embodiment, the processor 350 performing operations such as 'extracting', 'obtaining', and 'generating' may include the processor 350 directly performing the above-described operations by executing at least one instruction, as well as controlling other components such that the above-described operations are performed.

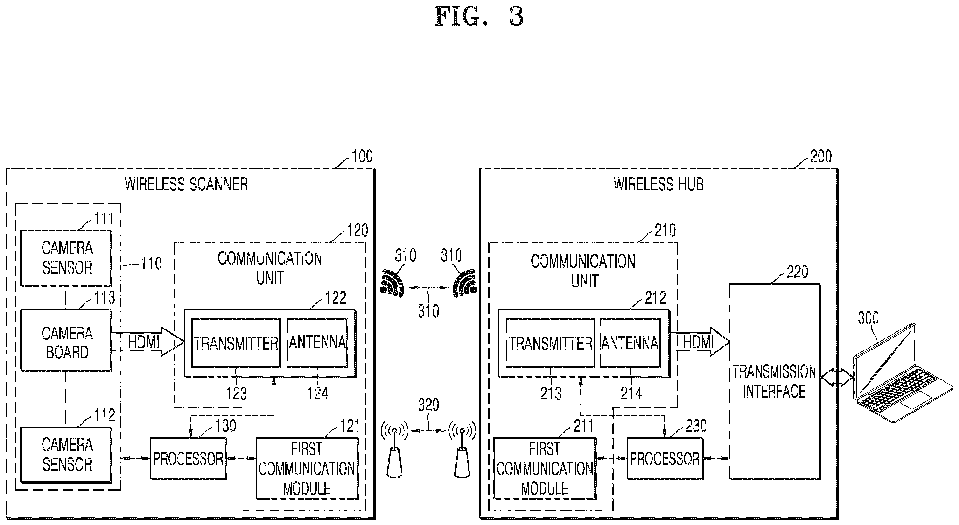

In order to implement embodiments of the present disclosure, the data processing device 100 may include only some of components shown in FIG. 3, or may include more components in addition to the components shown in FIG. 3.

In addition, the data processing device 300 may store and execute dedicated software linked to the wireless scanner 100. Here, the dedicated software may be referred to as a dedicated program, a dedicated tool, or a dedicated application. When the data processing device 300 operates in conjunction with the wireless scanner 100, the dedicated software stored in the data processing device 300 may be connected to the wireless scanner 100 to in real time receive data obtained by scanning the oral cavity.

The data processing device 300 may store and execute dedicated software corresponding to a wireless scanner product. The dedicated software may perform at least one operation for obtaining, processing, storing, and/or transmitting an intraoral image. Here, the dedicated software may be stored in the processor 350. In addition, the dedicated software may provide a user interface for using data obtained from the wireless scanner 100. Here, a user interface screen provided by the dedicated software may include the intraoral image generated according to the embodiment.

FIG. 3 is a detailed block diagram of the wireless scanner 100 and the wireless hub 200 according to an embodiment. In describing the configuration shown in FIG. 3, redundant descriptions with the above-described configurations are omitted.

Referring to FIG. 3, the wireless scanner 100 may include the optical unit 110, the communication unit 120 including the first communication module 121, and the second communication module 122, and the processor 130.

The optical unit 110 may include camera sensors 111 and 112 and a camera board 113.

The camera sensors 111 and 112 include at least one image sensor. Specifically, each of one or more cameras included in the camera sensors 111 and 112 may include a lens and an image sensor. Here, the image sensor may be a device that converts light entering the lens into an electrical signal to display an image, in order to obtain the image.

The camera board 113 may control the camera sensors 111 and 112 for image scanning. For example, the camera board 113 may set a region of interest (ROI), an exposure time, and/or a frame rate of each of the camera sensors 111 and 112.

Alternatively, the camera board 113 may generate image data corresponding to one or more images obtained by the camera sensors 111 and 112. For example, the camera board 113 may generate the image data corresponding to one or more images obtained by the camera sensors 111 and 112 by converting formats of the one or more images.

For example, the camera board 113 may format a plurality of images obtained by the camera sensors 111 and 112 into frame images corresponding to a High-Definition Multimedia Interface (HDMI) format, and output the frame images. Here, the frame images generated by the formatting are data having the HDMI format, and thus may be referred to as 'HDMI data'. Specifically, the camera board 113 may perform a formatting operation under control by the processor 130. Here, the formatting may mean modifying the format of data without data loss. The HDMI data output from the camera board 113 may be transmitted to the second communication module 122 for transmission to the wireless hub 200. Subsequently, the second communication module 122 may convert the received HDMI data into a signal of a frequency band defined by a HDMI communication network and transmit the signal to the wireless hub 200.

The optical unit 110 may further include a light emission unit (not shown) that outputs light to an object, and may obtain an image by performing an image scan on the object to which the light is emitted.

The optical unit 110 may perform scanning by using an optical triangulation method or a confocal method. The optical triangulation method is a scanning technique in which a light source (e.g., a pattern beam, etc.) is emitted toward an object to be scanned, and 3D depth information is obtained by using the light source reflected from the object. The a confocal method is a scanning technique in which, when light output from the light emission unit is reflected from an object, only information about light that is in focus is detected from among light reflected from the object to generate an image.

The communication unit 120 may include the first communication module 121 and the second communication module 122. The first communication module 121 may include a radio frequency (RF) antenna to perform bi-directional wireless communication with the wireless hub 200. That is, the first communication module 121 may be responsible for transmission and reception of control signals through a first communication network. Specifically, the first communication module 121 may transmit and receive a wireless signal having a frequency band lower than a second frequency band, specifically, an RF signal corresponding to a control signal. For example, the first communication module 121 may include an antenna that converts a control signal into a wireless signal of a frequency band such as 2.4 GHz or 5 GHz and outputs the wireless signal.

According to an embodiment, the first communication module 121 may receive a control signal from the wireless hub 200. Specifically, the first communication module 121 may receive a control signal for setting or controlling a camera of the wireless scanner 300 from the wireless hub 200.

According to an embodiment, the first communication module 121 may transmit a control signal to the wireless hub 200. Specifically, the first communication module 121 may transmit a control signal corresponding to state information indicating a state of the wireless scanner 100 to the wireless hub 200. For example, the state information of the wireless scanner 100 may include scanner mode information indicating an operation mode of the wireless scanner 100. The scanner mode information may include at least one of a scan mode, an idle mode, a deep sleep mode, or an off mode.

In addition, the second communication module 122 may perform uni-directional wireless communication. Specifically, the second communication module 122 may include a transmitter 123 and an antenna 124. The transmitter 123 may generate and/or process a signal to be transmitted. In addition, the antenna 124 may output the signal generated by the transmitter 123, as a radio signal (or a wireless signal). Alternatively, the second communication module 122 may perform bi-directional wireless communication.

In addition, the transmitter 123 may beamform the image data received from the camera board 113 as a radio signal of the second frequency band, for example, a frequency band of 60 GHz. Also, the antenna 124 may output the radio signal beamformed by the transmitter 123. Here, the outputting of the radio signal by the antenna 124 may mean radiating the radio signal toward the wireless hub 200 which is a receiving end.

Specifically, the transmitter 123 may process the radio signal to be output through the antenna 124 to have a certain phase and frequency. Specifically, the transmitter 123 may perform transmit (Tx) beamforming to generate a radio signal having a certain gain, phase, and frequency. Specifically, beamforming may refer to an operation of adjusting the overall radiation direction of a beam by adjusting the phase of a signal to be radiated through the antenna 124.

The antenna 124 may output the radio signal generated by the transmitter 123. Specifically, the antenna 124 may include an antenna array (not shown) including a plurality of antenna elements. The antenna array (not shown) may output a radio signal that directs a beam in a certain direction.

The second communication module 122 may perform uni-directional wireless communication from the wireless scanner 100 to the wireless hub 200 in a 60-GHz frequency band for fast transmission of image data. In this case, the transmitter 123 of the second communication module 122 may perform Tx beamforming for wireless Tx, and the antenna 124 may be a transmit antenna.

As in the above example, in a case in which the second communication module 122 is responsible for only data transmission, a transmission operation of communication module is not delayed due to a reception operation performed by one communication module. Accordingly, the second communication module 122 may rapidly transmit image data to the wireless hub 200 without a delay, by performing uni-directional wireless communication for only transmission. In addition, the first communication module 121 may perform bi-directional wireless communication such that a control signal necessary for controlling the wireless scanner 100 may be transmitted or received at any time regardless of transmission of image data. Accordingly, the transmission efficiency of the wireless scanner 100 may be increased, and the safety of a control operation may be improved by immediate transmission and reception of control signals.

The wireless hub 200 may include the communication unit 210, the transmission interface 220, and the processor 230.

The communication unit 210 may include the first communication module 211 and the second communication module 212.

The first communication module 211 may be responsible for communication through a first communication network 320. Specifically, the first communication module 211 may perform an operation corresponding to the first communication module 121 of the wireless scanner 100. For example, the first communication module 211 may transmit and receive an RF signal of a frequency band such as 2.4 GHz or 5 GHz, to and from the first communication module 121 of the wireless scanner 100.

The second communication module 213 may be responsible for communication through a second communication network. Specifically, the second communication module 213 may perform an operation corresponding to the second communication module 122 of the wireless scanner 100. For example, the second communication module 122 may receive a radio signal of a 60 GHz frequency band transmitted from the second communication module 122 of the wireless scanner 100.

Specifically, the second communication module 212 may include an antenna 213 and a receiver 214. The antenna 213 may receive a radio signal transmitted through a second communication network 310. In addition, the receiver 214 may generate and/or process a signal received by the antenna 213. In addition, in order to receive a radio signal, the receiver 214 may perform receive (Rx) beamforming to receive and process a radio wave corresponding to a certain phase and frequency. Here, a signal obtained by the RX beamforming by the receiver 214 of the second communication module 212 may be HDMI data.

The transmission interface 220 may perform an operation of transmitting a signal received from the communication unit 210 to the data processing device 300.

In addition, when HDMI data is transmitted from the second communication module 122 of the wireless scanner 100, the transmission interface 220 may receive the HDMI data conforming to an HDMI format and obtain a frame image from the received HDMI data. Then, the transmission interface 220 may transmit the obtained image to the data processing device 300. Specifically, the transmission interface 220 may decode the received HDMI data to obtain a frame image before being formatted into the HDMI data by the wireless scanner 100.

The processor 230 may execute at least one instruction to control the overall operation of the communication unit 210.

### Location of second communication module and structure of wireless hub in wireless scanner and wireless hub

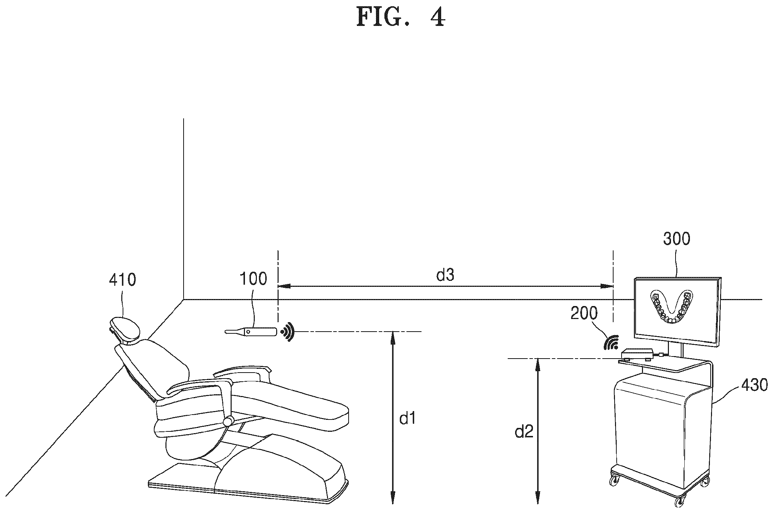

FIG. 4 illustrates an example in which a system including the wireless scanner 100, the wireless hub 200, and the data processing device 300 is arranged in an actual use environment.

Referring to FIG. 4, a patient 420 is lying on a treatment chair 410, and the wireless scanner 100 may be used at a location d1 suitable for scanning an oral cavity of the patient 420 lying on the treatment chair 410. In order for a user, such as a doctor, to input data into the data processing device 300, the data processing device 300 may be placed on a desk 430 having a constant height d2, and the wireless hub 200 may be placed at an appropriate location, for example, the desk 430, for mediating between the wireless scanner 100 and the data processing device 300. In addition, the wireless hub 200 and the wireless scanner 100 may be used within an appropriate distance d3 for signal transmission and reception.

In order to transmit image data from the wireless scanner 100 to the wireless hub 200 and receive image data by the wireless hub 200 from the wireless scanner 100, as described above through FIGS. 2 and 3, a second communication module that communicates a wireless signal of a second frequency band, for example, 60 GHz, may be used. Because the wireless signal of the second frequency band used in the second communication module has a stronger straightness as the frequency increases, a data transmission speed is fast but a transmission distance is short. Therefore, in order to obtain optimal image transmission/reception efficiency through the second communication module, it may be considered that the second communication module is disposed in the optimal location within the wireless scanner 100 and the wireless hub 200 in consideration of a usage environment or device characteristics. According to an example, the second communication module 122 of the wireless scanner 100 and the second communication module 212 in the wireless hub 200 may be preferably located to face each other to improve image transmission and reception efficiency.

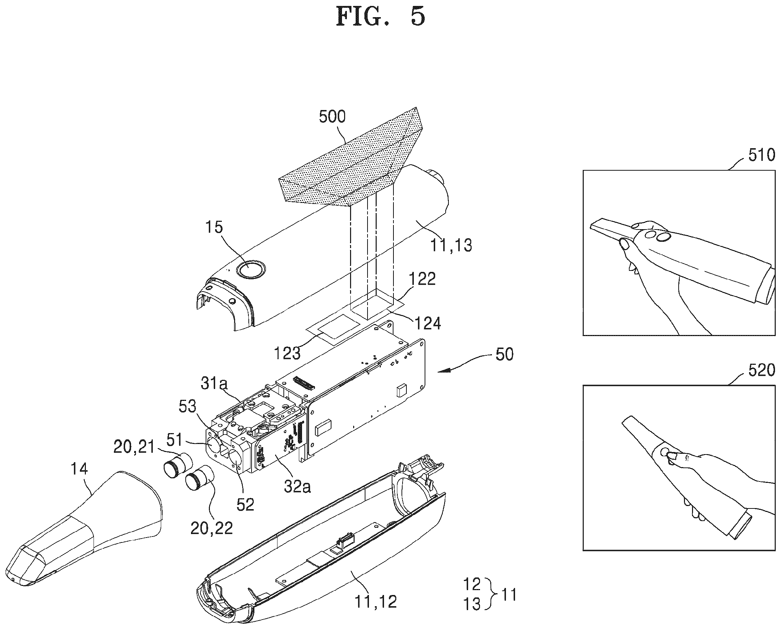

Hereinafter, an appropriate location of the second communication module 122 disposed in the wireless scanner 100 and an appropriate location of the second communication module 212 disposed in the wireless hub 200 will be described with reference to FIGS. 5 to 8.

FIG. 5 is a reference diagram for explaining an appropriate location of the second communication module 122 disposed in the wireless scanner 100 according to an embodiment.

Referring to FIG. 5, the wireless scanner 100 includes a main body case 11 including a lower case 12 having a certain space in which components of the wireless scanner 100 are embedded, and an upper case 13 detachably coupled to the lower case 12 to cover the components.

The wireless scanner 100 may further include a tip case 14 coupled to one end of the main body case 11 and having an input and output light path unit including an opening to guide light incident into the main body case 11 through the opening and light emitted from the inside of the main body case 11 through the opening. One end of a pair of lenses 20 may be provided to protrude toward the tip case 14, the other end of the pair of lenses 20 may be inserted and installed, and a camera mounting unit 50 may be disposed to form an optical waveguide, which is a path of incident light transmitted through the pair of lenses 20 or emitted light emitted from a light projector. The optical waveguide may include an emitted light path unit 53 that provides an optical path of the emitted light emitted from the light projector to the tip case 14, one side incident light path unit 51 that provides an optical path of incident light incident through one lens of the pair of lenses 20, and other side incident light path unit 52on the other side that provides an optical path of incident light incident through the other lens of the pair of lenses 20.

The light projector is located at the center of the other end of the pair of lenses 20 spaced apart from each other by a certain distance in a width direction of the main body case 11, and the emitted light path unit 53 may be formed between the one side incident light path unit 51 and the other side incident light path unit 52.

The one side incident light path unit 51 and the other side incident light path unit 52 are formed to correspond to longitudinal directions of the respective corresponding lenses such that incident light from the pair of lenses 20 is transmitted, but may be formed to be opened to one side and the other side of the camera mounting unit 50, respectively.

Here, imaging boards 31a and 32a into which imaging sensors (not shown) are integrated may be disposed vertically so as to be in close contact with one side wall of the main body case 11 in a width direction and the other side wall in the width direction. More specifically, the imaging board 31a on one side may be disposed to be in close contact with one side surface of the camera mounting unit 50, but may be disposed between one side wall of the main body case 11 in the width direction. In addition, the imaging board 32a on the other side may be disposed between the other side wall of the main body case 11 in the width direction so as to be in close contact with the other side surface of the camera mounting unit 50. At this time, the imaging board 31a on one side may be provided so that the imaging sensor integrated into the imaging board 31a is exposed to the one side incident light path unit 51, and the imaging board 32a on the other side may be provided so that the imaging sensor integrated into the imaging board 32a is exposed to the other side incident light path unit 52.

The wireless scanner 100 may further include a pair of optical path changing units (not shown) disposed to change the path of incident light passing through the pair of lenses 20, respectively, toward the imaging sensors integrated into the imaging boards 31a and 32a.

Image data generated by the imaging boards 31a and 32a may be transmitted to the second communication module 122. The transmitter 123 of the second communication module 122 may receive and beamform the image data generated by the imaging boards 31a and 32a as a radio signal of a second frequency band, for example, a frequency band of 60 GHz. Also, the antenna 124 may output the radio signal beamformed by the transmitter 123. Here, the outputting of the radio signal by the antenna 124 may mean radiating the radio signal toward the wireless hub 200 which is a receiving end. A radiation region 500 may be preferably generated to allow the radio signal output from the antenna 124 to be effectively transmitted to the wireless hub 200. The antenna 124 may be preferably disposed on an upper end of the rear surface of the wireless scanner 100 so that the radiation area 500 may be generated to allow the radio signal output from the antenna 124 to be effectively transmitted to the wireless hub 200.

In general, considering the form in which the wireless scanner 100 is located and the form in which the wireless scanner 100 is gripped when users operate the wireless scanner 100, the antenna 124 may be preferably disposed on the top of the rear surface of the wireless scanner 100. In other words, referring to FIG. 5, because the optical unit is located in the front of the main body of the wireless scanner 100 due to a structure of the wireless scanner 100, the user may operate the wireless scanner 100 in the form 510 gripping a tip part of the wireless scanner 100 like a pencil or in the form 520 gripping a body part of the wireless scanner 100. Accordingly, a module for wireless communication may be disposed on the rear surface of the wireless scanner 100. That is, a wireless signal emitted from the second communication module 122 for communication with the wireless hub 200 may be preferably transmitted at the back of the wireless scanner 100. In addition, the second communication module 122 may be disposed on one of upper, lower, and side portions of the rear surface of the main body of the wireless scanner 100. According to an example, the second communication module 122 including the antenna 124 may be preferably disposed on the upper end of the rear surface of the wireless scanner 100. As described above, the second communication module 122 may be disposed in the wireless scanner 100 so that the radiation region 500 of the wireless signal irradiated from the antenna 124 is formed on the upper case 13 of the wireless scanner 100, and thus, the irradiation of the wireless signal may not be prevented even in various forms in which the user gripping the wireless scanner 100.

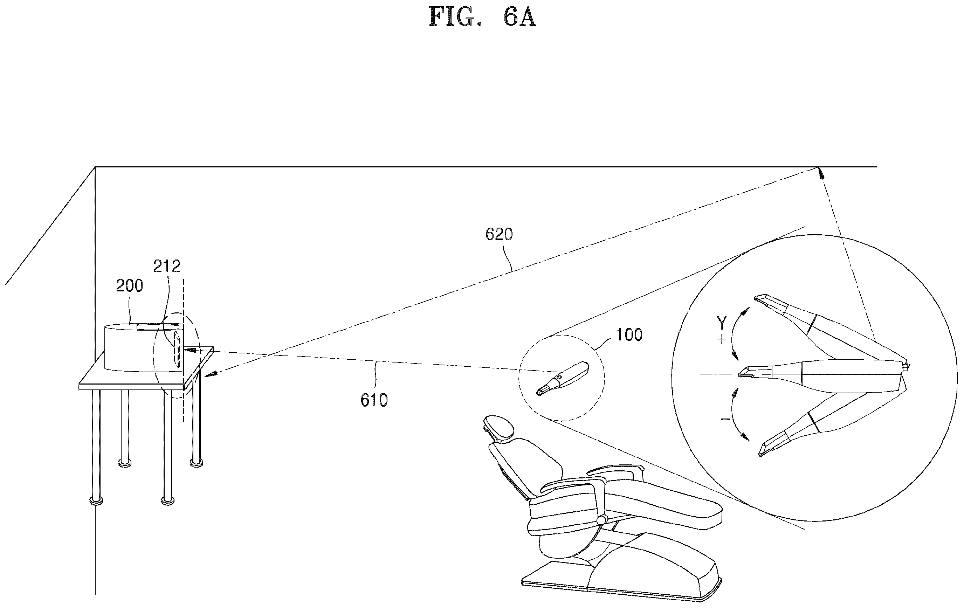

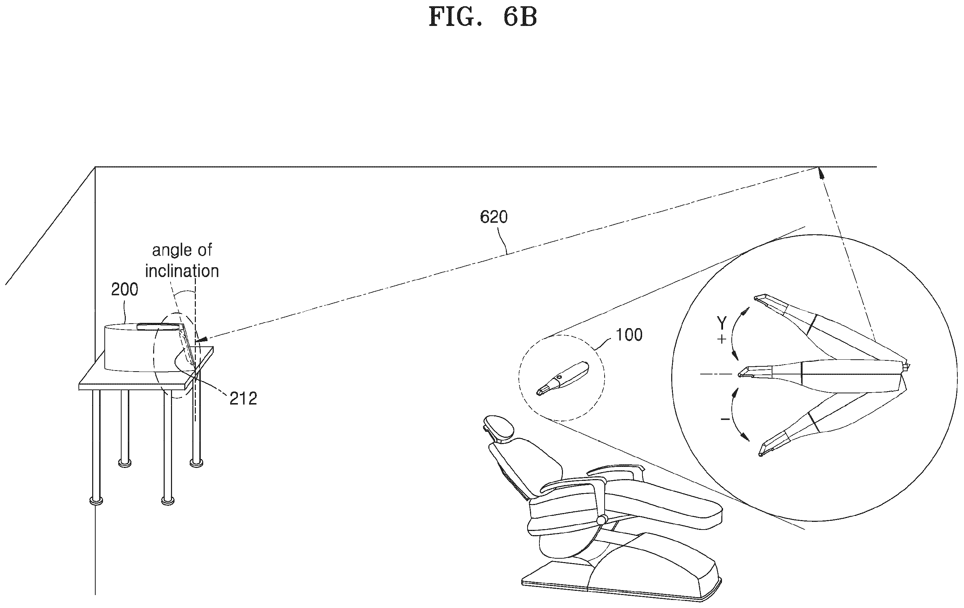

FIGS. 6A and 6B are reference diagrams for explaining an appropriate location of the second communication module 212 disposed in the wireless hub 200 according to an embodiment.

Referring to FIG. 6A, when a patient lies in a chair for patient and opens his or her mouth in consideration of a dental treatment environment, a user may grip and manipulate the wireless scanner 100 at a location where the wireless scanner 100 may be inserted into a patient's oral cavity. Also, the wireless hub 200 that receives a wireless signal emitted from the wireless scanner 100 may be used while being placed on a cradle at a certain height.

At this time, a location, direction, and inclination of the second communication module 212 disposed within the wireless hub 200 may be preferably determined to most efficiently receive the wireless signal emitted from the wireless scanner 100 by considering the location where the wireless hub 200 is placed and the direction of an antenna of the wireless scanner 100.

First, in order to facilitate signal transmission and reception between the antenna of the wireless scanner 100 and an antenna of the wireless hub 200, the antenna of the wireless scanner 100 and the antenna of the wireless hub 200 may be preferably disposed to face each other. Therefore, the wireless hub 200 may be preferably disposed such that the antenna disposed in the wireless hub 200 faces the wireless scanner 100.

The antenna of the wireless scanner 100 may employ beamforming technology, and thus, as shown in FIG. 6A, a wireless signal 620 may allow the radio signal emitted from the wireless scanner 100 to be incident directly toward the antenna of the wireless hub 200, like line 610. However, there may be various obstacles due to the nature of a dental treatment space, and it may also be better for the radio signal emitted from the wireless scanner 100 to enter the wireless hub 200 through a reflection path through the ceiling or the wall. Therefore, it may preferably find the most optimal path considering such various situations.

Referring to FIG. 6A, the second communication module 212 disposed in the wireless hub 200 is disposed perpendicular to the ground where the wireless hub 200 is installed, that is, at an inclination of 0 °. In this case, when the wireless signal emitted from the wireless scanner 100 is directly incident on the second communication module 212 within the wireless hub 200, the signal reception efficiency may be increased. However, when the user moves the wireless scanner 100 in the + direction, the wireless signal emitted from the wireless scanner 100 hits the ceiling, and in this case, not all wireless signals 620 are incident on the second communication module 212 of the wireless hub 200, and thus, the reception efficiency may be reduced.

Accordingly, in order to solve this problem, in FIG. 6B, the second communication module 212 disposed in the wireless hub 200 is inclined at a certain angle of inclination on the side wall of the wireless hub 200, and thus, the reception efficiency may be increased even when the user moves and uses the wireless scanner 100 in the + direction or the - direction.

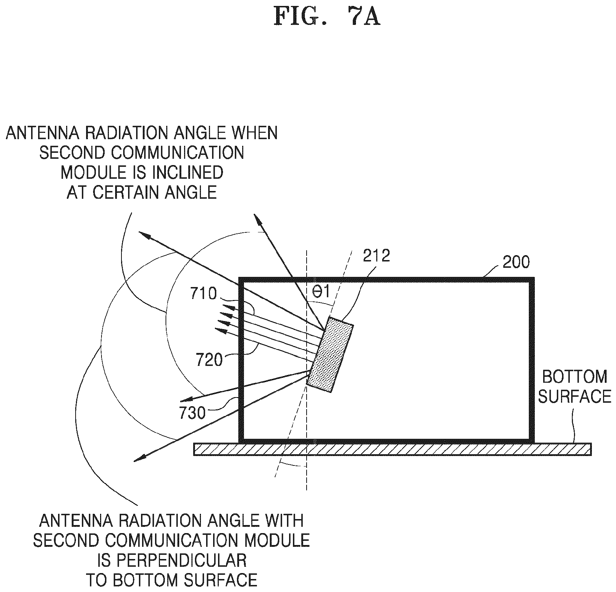

FIG. 7A illustrates an example in which the second communication module 212 is disposed to be inclined at a certain angle in the wireless hub 200 according to an embodiment.

Referring to FIG. 7A, the second communication module 212 may be disposed on the wireless hub 200 to be inclined at a certain angle θ1 with a surface perpendicular to a bottom surface of the wireless hub 200. As described above, the second communication module 212 is inclined by a certain angle θ1 rather than perpendicular to the bottom surface, and thus, an antenna radiation angle of the second communication module 212 may be directed upward by the certain angle θ1.

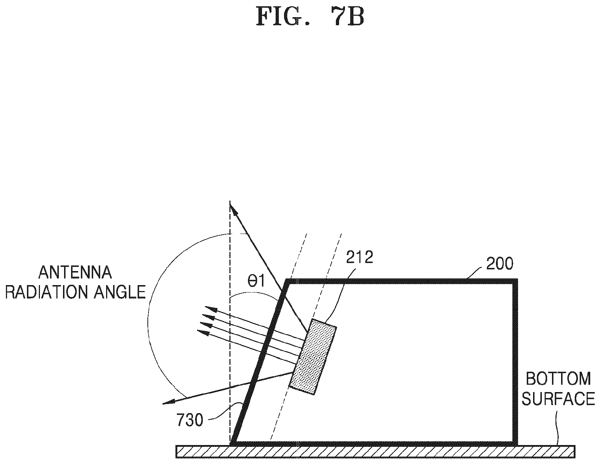

FIG. 7B illustrates another example in which the second communication module 212 is disposed to be inclined at the certain angle θ1 in the wireless hub 200 according to an embodiment.

FIG. 7B is the same as FIG. 7A in that the second communication module 212 is disposed to be inclined at a certain angle in the wireless hub 200, but is different from FIG. 7A in a sidewall 730 of the wireless hub 200 through which a wireless signal emitted from the second communication module 212 passes.

In FIG. 7A, because the sidewall 730 of the wireless hub 200 is perpendicular to the bottom surface on which the wireless hub 200 is disposed, distances between wireless signals emitted from the antenna of the second communication module 212 passing through the sidewall 730 may vary. That is, a distance from a wireless signal 710 to the sidewall 730 of the wireless hub 200 and a distance from a wireless signal 720 to the sidewall 730 of the wireless hub 200 are different, and thus, signal efficiency may be reduced. That is, when the sidewall 730 of the wireless hub 200 is not parallel to the second communication module 212, diffuse reflection may occur, and a propagation sensitivity function may be deteriorated.

In FIG. 7B, to solve this problem, the sidewall 730 of the wireless hub 200 through which the wireless signal emitted from the second communication module 212 passes may be formed to have the same inclination as the inclined angle θ1 of the second communication module 212, and thus, the signal efficiency may be increased.

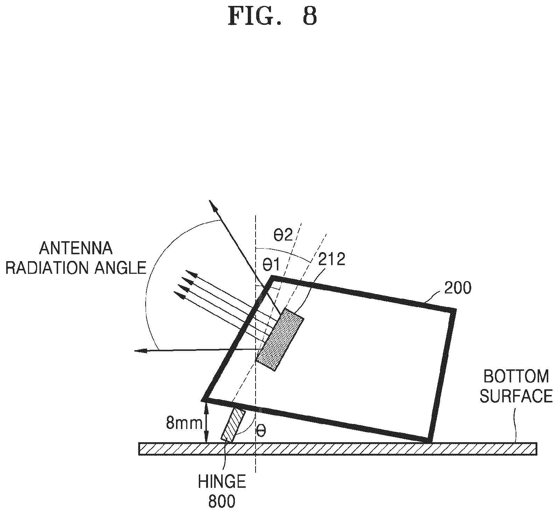

FIG. 8 illustrates another example in which the second communication module 212 is disposed to be inclined at a certain angle θ2 in the wireless hub 200 according to an embodiment.

In FIG. 8, as shown in FIG. 7B, the second communication module 212 is disposed to be inclined at a certain angle within the wireless hub 200, and the sidewall 730 of the wireless hub 200 through which a wireless signal of the second communication module 212 passes is formed to be inclined at the same certain angle so that the wireless signal from the second communication module 212 moves by a uniform distance to pass through the sidewall 730. In FIG. 8, a structure of a hinge 800 may be adopted at a lower end of the wireless hub 200 to more flexibly adjust the angle at which the second communication module 212 is inclined.

Due to the structure of the hinge 800, the angle θ2 at which the second communication module 212 is inclined may be greater than the angle θ1 at which the second communication module 212 is inclined in FIG. 7B in which a hinge structure is not adopted.

According to an embodiment, the hinge 800 disposed at the lower end of the wireless hub 200 is provided to adjust its inclined angle θ so that the angle at which the wireless hub 200 is inclined from the bottom surface may be adjusted. The hinge 800 may be adjusted to increase the angle θ between the hinge 800 and the wireless hub 200, so that the angle of the second communication module 212 of the wireless hub 200 is inclined may be reduced, and the hinge 800 may be adjusted to reduce the angle θ between the hinge 800 and the wireless hub 200, so that the angle of the second communication module 212 of the wireless hub 200 is inclined may be increased.

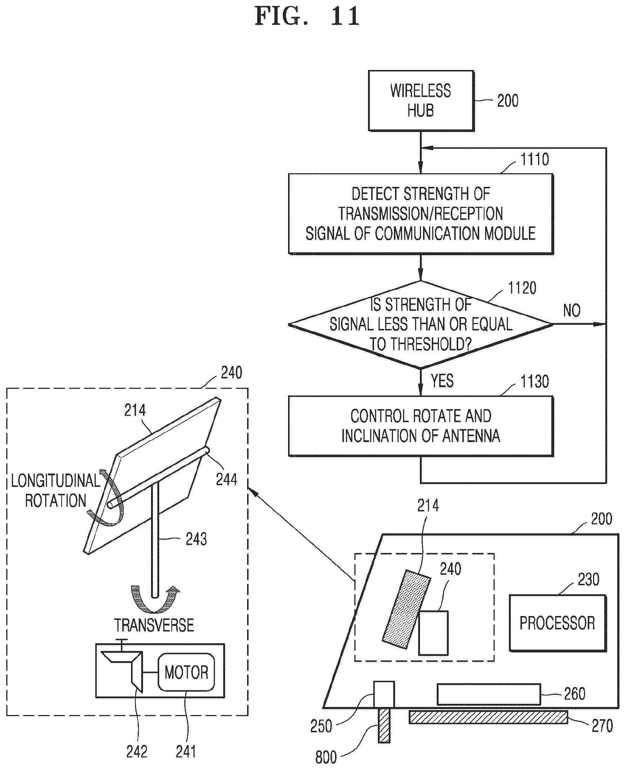

According to an embodiment, the hinge 800 of the wireless hub 200 may be manually or automatically adjusted. Specifically, the inclination of the hinge 800 of the wireless hub 200 may be manually adjusted by the manipulation of the user, or automatically adjusted by electronic and/or mechanical means of the wireless hub 200. A configuration automatically adjusting the hinge 800 of the wireless hub 200 will be described below with reference to FIG. 11.

According to an embodiment, the wireless hub 200 may provide a guide for guiding to adjust the angle of the hinge 800 installed in the wireless hub 200 according to the low signal reception efficiency of the wireless hub 200.

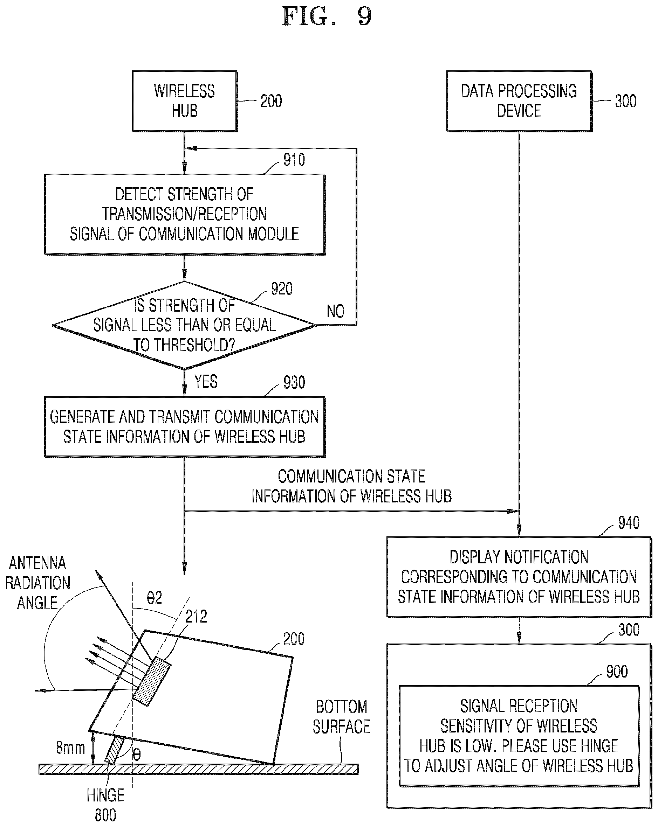

FIG. 9 is a flowchart illustrating an operation of providing a guide to a user by transmitting state information of the wireless hub 200 to the data processing device 300 according to an embodiment.

Referring to FIG. 9, in operation 910, the wireless hub 200 may detect the strength of a transmission/reception signal of a communication module. Specifically, the processor 230 of the wireless hub 200 may detect the strength of a transmission/reception signal of the second communication module 212. For example, the second communication module 212 may provide information about the strength of the transmission/reception signal to the processor 230.

In operation 920, the wireless hub 200 may determine whether the detected strength of the transmission/reception signal is less than or equal to a threshold. Specifically, when it is determined that the detected strength of the transmission/reception signal is not less than or equal to the threshold, the processor 230 of the wireless hub 200 may proceed to operation 910 to continuously detect the strength of the transmission/reception signal. When it is determined that the detected strength of the transmission/reception signal is less than or equal to the threshold, the processor 230 of the wireless hub 200 may proceed to operation 930.

In operation 930, the wireless hub 200 may generate and transmit communication state information of the wireless hub 200. Specifically, the processor 230 of the wireless hub 200 may control a communication unit to generate communication state information corresponding to the detected strength of the transmission/reception signal and transmit the generated communication state information to the data processing device 300 through wired/wireless communication. As wireless communication, short-range communication such as Bluetooth communication, Wi-Fi communication, and NFC communication may be used. A cable may be used as wired communication. The generated communication state information may include, for example, the information about the strength of the transmission/reception signal or information indicating that the communication state is poor.

In operation 940, the data processing device 300 may display a notification corresponding to the communication state information of the wireless hub 200 transmitted from the wireless hub 200. Specifically, a notification message to be provided corresponding to the communication state information of the wireless hub 200 transmitted from the wireless hub 200 may be stored in a memory of the data processing device 300. The processor 350 of the data processing device 300 may check the communication state information of the wireless hub 200 transmitted from the wireless hub 200 and obtain the notification message corresponding to the communication state information of the wireless hub 200. Also, the processor 230 may display the obtained notification message on a display. For example, as shown in FIG. 9, the processor 350 may display a notification message 900 <The signal reception sensitivity of the wireless hub is low. Please use the hinge to adjust the angle of the wireless hub> on the display 320.

As described above, the processor 350 may check the notification message 900 displayed on the display 320, and a user may manually adjust the angle of the wireless hub 200 by using the hinge 800 of the wireless hub 200.

FIG. 9 shows that, according to an example, the wireless hub 200 transmits the communication state information of the wireless hub 200 to the data processing device 300 so that the data processing device 300 displays the notification message, thereby providing notification information to the user. However, embodiments are not limited thereto, and a notification may be provided to the user according to various methods. For example, the data processing device 300 may receive the communication state information of the wireless hub 200 and provide a notification message corresponding thereto by voice. For example, the wireless hub 200 may provide the notification message corresponding to the communication state information of the wireless hub 200 by itself. For example, the wireless hub 200 may provide the notification message corresponding to the communication state information of the wireless hub 200 by voice, display the notification message through the display to display the notification message, or output the notification message through vibration or LED.

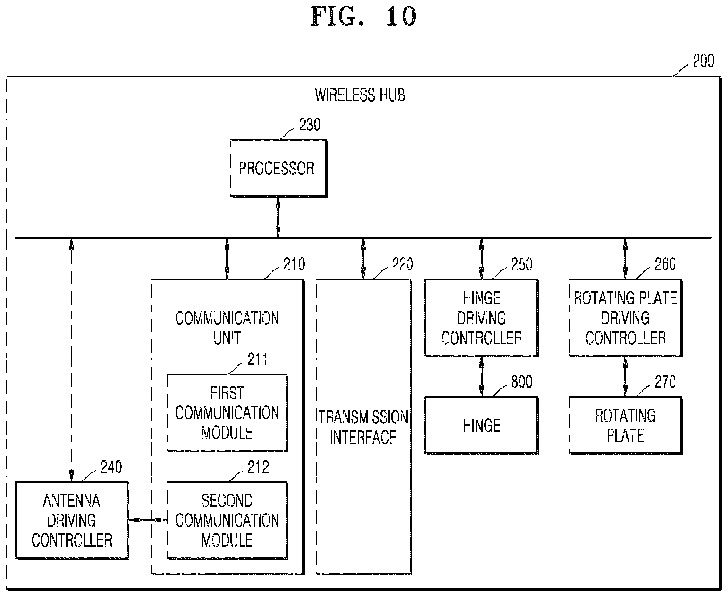

FIG. 10 illustrates an example of the wireless hub 200 that enables automatic adjustment of a radiation region of the second communication module 212 according to communication state information of the wireless hub 200 according to an embodiment.

Referring to FIG. 10, in addition to components of the wireless hub 200 shown in FIG. 2, that is, the communication unit 210, the transmission interface 220, and the processor 230, the wireless hub 200 may further include an antenna driving controller 240, a hinge driving controller 250, the hinge 800, a rotating plate driving controller 260, and a rotating plate 270. According to the embodiments, the wireless hub 200 does not need to include all of the antenna driving controller 240, the hinge driving controller 250, the hinge 800, the rotating plate driving controller 260, and the rotating plate 270, but may include one or more of these components.

The antenna driving controller 240 may control the rotation or inclination of the antenna 214 of the second communication module 212 by the control of the processor 230.

The hinge driving controller 250 may control the inclination of the hinge 800 by the control of the processor 230.

The rotating plate 270 may be attached to the bottom surface of the wireless hub 200 and operate to be rotatable.

The rotating plate driving controller 260 may control the rotation angle of the rotating plate 270 by the control of the processor 230.

The processor 230 may monitor transmission/reception signal strength information of the second communication module 212 and perform an operation of automatically adjusting the radiation region of the antenna 214 of the second communication module 212 when the strength of a transmission/reception signal falls below a threshold. Specifically, the processor 230 may perform an operation of controlling the rotation or inclination of the antenna 214 of the second communication module 212 or controlling the inclination of the hinge 800 to adjust the radiation region of the antenna 214 of the second communication module 212.

According to an embodiment, the processor 230 may provide, to the antenna driving controller 240, a command to adjust the rotation or inclination of the antenna 214 so as to control the rotation or inclination of the antenna 214 of the second communication module 212. Specifically, the processor 230 may provide, to the antenna driving controller 240, the command to adjust the rotation or inclination of the antenna 214 by a predetermined rotation increment amount or a predetermined inclination increment amount with respect to the antenna 214. For example, the processor 230 may control the rotation or inclination of the antenna 214 according to a predetermined rotation increment amount of 1 degree or a predetermined inclination increment amount of 1 degree, detect the signal transmission/reception strength in the state of the antenna 214 rotated or inclined by the increased amount to determine whether a threshold is satisfied, and provide, to the antenna driving controller 240, the command to adjust the rotation or inclination of the antenna 214 by the predetermined rotation increment amount or the predetermined inclination increment amount with respect to the antenna 214 until the threshold is satisfied. The antenna driving controller 240 may control the rotation or inclination of the antenna 214 according to the received command.

According to an embodiment, the processor 230 may provide, to the antenna driving controller 240, a command to adjust the inclination of the hinge 800 so as to control the inclination of the hinge 800. Specifically, the processor 230 may provide, to the hinge driving controller 250, the command to adjust the inclination of the hinge 800 by a predetermined inclination increment amount with respect to the hinge 800. For example, the processor 230 may control the inclination of the hinge 800 according to a predetermined rotation increment amount of 1 degree or a predetermined inclination increment amount of 1 degree, detect the signal transmission/reception strength in the state of the hinge 800 inclined by the increased amount to determine whether a threshold is satisfied, and provide, to the hinge driving controller 250, the command to adjust the inclination of the hinge 800 by the predetermined inclination increment amount with respect to the hinge 800 until the threshold is satisfied. The hinge driving controller 250 may control the inclination of the hinge 800 according to the received command.

According to an embodiment, the processor 230 may provide, to the rotation angle driving controller 260, a command to adjust a rotation angle of the rotating plate 270 so as to control the rotation angle of the rotating plate 270. Specifically, the processor 230 may provide, to the rotating plate driving controller 270, the command to adjust the rotation angle of the rotating plate 270 by a predetermined rotation increment amount with respect to the rotating plate 270. For example, the processor 230 may control the rotation amount of the rotating plate 270 according to a predetermined rotation increment of 1 degree, detect the signal transmission/reception strength in the state of the rotating plate 270 rotated by the increased amount to determine whether a threshold is satisfied, and provide, to the rotation angle driving controller 260, a command to adjust the rotation amount of the rotating plate 270 by the predetermined rotation increment amount with respect to the rotating plate 270 until the threshold is satisfied. The rotating plate driving controller 260 may control the rotation amount of the rotating plate 270 according to the received command.

FIG. 11 is a reference diagram for explaining an operation of the wireless hub 200 that enables automatic adjustment of rotation or inclination of the antenna 214 of the second communication module 212 according to communication state information of the wireless hub 200 according to an embodiment.

Referring to FIG. 11, the antenna driving controller 240 may include a motor 241, a gear 242, a rotation support 243 performing transverse rotation, and a tilt support 244 performing longitudinal rotation.

The antenna 214 may be rotated variously according to angles at which the rotation support 243 and the tilt support 244 rotate.

The rotation support 243 may be connected to the antenna 214 to rotate the antenna 214 by a certain angle. For example, when a range in which the rotation support 243 rotates in the transverse direction is 120 degrees, the transverse range of the antenna 214 may be adjusted within 120 degrees according to the rotation range of the rotation support 243.

The tilt support 244 may be connected to the antenna 214 to rotate, i.e., to be inclined, the antenna 214 in the longitudinal direction by a certain angle. For example, when a range in which the tilt support 244 rotates in the longitudinal direction is 60 degrees, the longitudinal range, that is, inclination, of the antenna 214 may be adjusted within 60 degrees according to the rotation range of the tilt support 244. According to an embodiment, the volume of the product may be significantly reduced by making the antenna 214, the motor 241, the gear 242, the rotation support 243, and the tilt support 244 ultra-small with a micro electro-mechanical systems (MEMS) technology.

An operation of the wireless hub 200 including the antenna driving controller 240 will be described.

In operation 1110, the wireless hub 200 may detect the strength of a transmission/reception signal of a communication module. Specifically, the processor 230 of the wireless hub 200 may detect the strength of a transmission/reception signal of the second communication module 212. For example, the second communication module 212 may provide information about the strength of the transmission/reception signal to the processor 230.

In operation 1120, the wireless hub 200 may determine whether the detected strength of the transmission/reception signal is less than or equal to a threshold. Specifically, when it is determined that the detected strength of the transmission/reception signal is not less than or equal to the threshold, the processor 230 of the wireless hub 200 may proceed to operation 1110 to continuously detect the strength of the transmission/reception signal. When it is determined that the detected strength of the transmission/reception signal is less than or equal to the threshold, the processor 230 of the wireless hub 200 may proceed to operation 1130.

In operation 1130, the wireless hub 200 may control at least one of rotation or inclination of the antenna 214. Specifically, the processor 230 may provide, to the antenna driving controller 240, a command to adjust the rotation or inclination of the antenna 214 so as to control the rotation or inclination of the antenna 214 of the second communication module 212. Specifically, the processor 230 may provide, to the antenna driving controller 240, the command to adjust the rotation or inclination of the antenna 214 by a predetermined rotation increment amount or a predetermined inclination increment amount with respect to the antenna 214. For example, when the processor 230 provides, to the antenna driving controller 240, the command to adjust the rotation or inclination of the antenna 214 according to a predetermined rotation increment amount of 1 degree or a predetermined inclination increment amount of 1 degree, the antenna driving controller 240 may control the rotation or inclination of the antenna 214 by the increased amount according to the received command. As described above, the processor 230 may detect the strength of the transmission/reception signal of the second communication module 212 in the state of the antenna 214 rotated or inclined by the determined amount to determine whether a threshold is satisfied, and provide, to the antenna driving controller 240, the command to adjust the rotation or inclination of the antenna 214 by the predetermined rotation increment amount or the predetermined inclination increment amount with respect to the antenna 214 until the threshold is satisfied. By using such a method, the antenna 214 of the wireless hub 200 may be controlled to be rotated or inclined in a direction satisfying the strength of the transmission/reception signal.

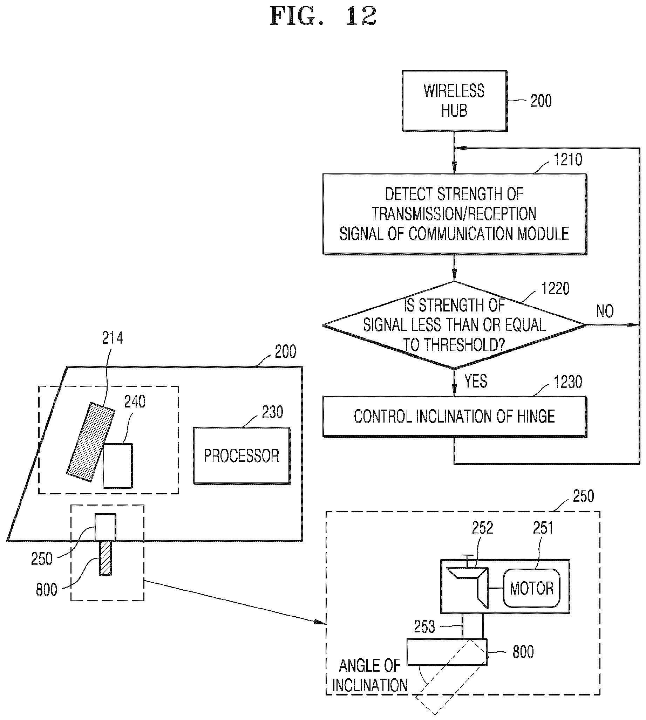

FIG. 12 is a reference diagram for explaining an operation of the wireless hub 200 that enables automatic adjustment of the hinge 800 installed in the wireless hub 200 according to communication state information of the wireless hub 200 according to an embodiment.

Referring to FIG. 12, the hinge driving controller 250 may include a motor 251, a gear 252, and a tilt support 253 for longitudinal rotation of the hinge 800.

The hinge 800 may rotate in the longitudinal direction according to an angle at which the tilt support 253 rotates.

The hinge 800 may be installed on a part of a bottom surface of the wireless hub 200 and rotate in the longitudinal direction by the control of the tilt support 253, causing the bottom surface of the wireless hub 200 to fall from the ground at a part where the hinge 800 is installed, and thus, the wireless hub 200 may be inclined. One side of the wireless hub 200 may be inclined to have an inclination angle, and the antenna 214 installed in the wireless hub 200 may be inclined, thereby adjusting a radiation region of a wireless signal emitted from the antenna 214.

An operation of the wireless hub 200 including the hinge driving controller 250 will be described.

In operation 1210, the wireless hub 200 may detect the strength of a transmission/reception signal of a communication module. Specifically, the processor 230 of the wireless hub 200 may detect the strength of a transmission/reception signal of the second communication module 212. For example, the second communication module 212 may provide information about the strength of the transmission/reception signal to the processor 230.

In operation 1220, the wireless hub 200 may determine whether the detected strength of the transmission/reception signal is less than or equal to a threshold. Specifically, when it is determined that the detected strength of the transmission/reception signal is not less than or equal to the threshold, the processor 230 of the wireless hub 200 may proceed to operation 1210 to continuously detect the strength of the transmission/reception signal. When it is determined that the detected strength of the transmission/reception signal is less than or equal to the threshold, the processor 230 of the wireless hub 200 may proceed to operation 1230.

In operation 1230, the wireless hub 200 may control inclination of the hinge 800. Specifically, the processor 230 may provide, to the hinge driving controller 250, a command to adjust the inclination of the hinge 800 so as to control the inclination of the hinge 800. Specifically, the processor 230 may provide, to the hinge driving controller 250, the command to adjust the inclination of the hinge 800 by a predetermined inclination increment amount with respect to the hinge 800. For example, when the processor 230 provides, to the hinge driving controller 250, the command to adjust the inclination of the hinge 800 according to a predetermined inclination increment amount of 1 degree, the hinge driving controller 250 may control the inclination of the hinge 800 by the increased amount according to the received command. As described above, the processor 230 may detect the strength of the transmission/reception signal of the second communication module 212 in the state of the hinge 800 inclined by the determined amount to determine whether a threshold is satisfied, and provide, to the hinge driving controller 250, the command to adjust the inclination of the hinge 800 by the predetermined inclination increment amount with respect to the hinge 800 until the threshold is satisfied. By using such a method, the wireless hub 200 may be controlled to be inclined in a direction satisfying the strength of the transmission/reception signal by controlling the inclination of the hinge 800 of the wireless hub 200.

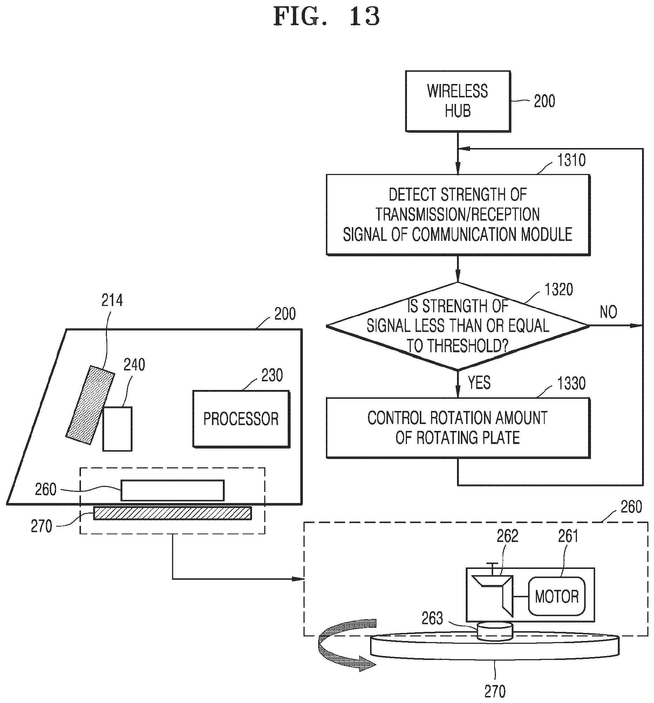

FIG. 13 is a reference diagram for explaining an operation of the wireless hub 200 that enables automatic adjustment of the rotating plate 270 installed in the wireless hub 200 according to communication state information of the wireless hub 200 according to an embodiment.

Referring to FIG. 13, the rotating plate driving controller 260 may include a motor 261, a gear 262, and a rotating plate support 263.

The rotating plate 270 may be attached to a bottom surface of the wireless hub 200 and may rotate according to an angle at which the rotating plate support 263 rotates.

According to the rotation of the rotation plate 270, a direction viewed by the antenna 214 of the second communication module 212 installed in the wireless hub 200 may vary, and accordingly, a radiation region of a wireless signal emitted by the antenna 214 may be controlled.

An operation of the wireless hub 200 including the rotating plate driving controller 260 will be described.

In operation 1310, the wireless hub 200 may detect the strength of a transmission/reception signal of a communication module. Specifically, the processor 230 of the wireless hub 200 may detect the strength of the transmission/reception signal of the second communication module 212. For example, the second communication module 212 may provide information about the strength of the transmission/reception signal to the processor 230.

In operation 1320, the wireless hub 200 may determine whether the detected strength of the transmission/reception signal is less than or equal to a threshold. Specifically, when it is determined that the detected strength of the transmission/reception signal is not less than or equal to the threshold, the processor 230 of the wireless hub 200 may proceed to operation 1310 to continuously detect the strength of the transmission/reception signal. When it is determined that the detected strength of the transmission/reception signal is less than or equal to the threshold, the processor 230 of the wireless hub 200 may proceed to operation 1330.

In operation 1330, the wireless hub 200 may control the rotation of the rotating plate 270. Specifically, the processor 230 may provide a command to adjust the rotation amount of the rotating plate 270 to the rotating plate driving controller 260 so as to control the rotation of the rotating plate 270. Specifically, the processor 230 may provide, to the rotating plate driving controller 260, the command to adjust the rotation amount of the rotating plate 270 by a predetermined rotation increment amount with respect to the rotating plate 270. For example, when the processor 230 provides, to the rotating plate driving controller 260, the command to adjust the rotation amount according to a predetermined rotation increment amount of 1 degree, the rotating plate driving controller 260 may control the rotation of the rotating plate 270 by the increased amount according to the received command. As described above, the processor 230 may detect the strength of the transmission/reception signal of the second communication module 212 in the state of the wireless hub 200 rotated by the determined amount to determine whether a threshold is satisfied, and provide, to the rotating plate driving controller 260, the command to adjust the rotation of the rotating plate 270 by the predetermined rotation increment amount with respect to the rotating plate 270 until the threshold is satisfied. By using such a method, the wireless hub 200 may be controlled to be rotated in a direction satisfying the strength of the transmission/reception signal by controlling a direction in which the antenna 214 of the wireless hub 200 is viewed by using the rotating plate 270 of the wireless hub 200.

Although antenna control, hinge control, and rotation plate control have been described above with reference to FIGS. 11 to 13, the wireless hub 200 according to embodiments may be implemented by adopting antenna control, hinge control, and rotation plate control, or a combination thereof.

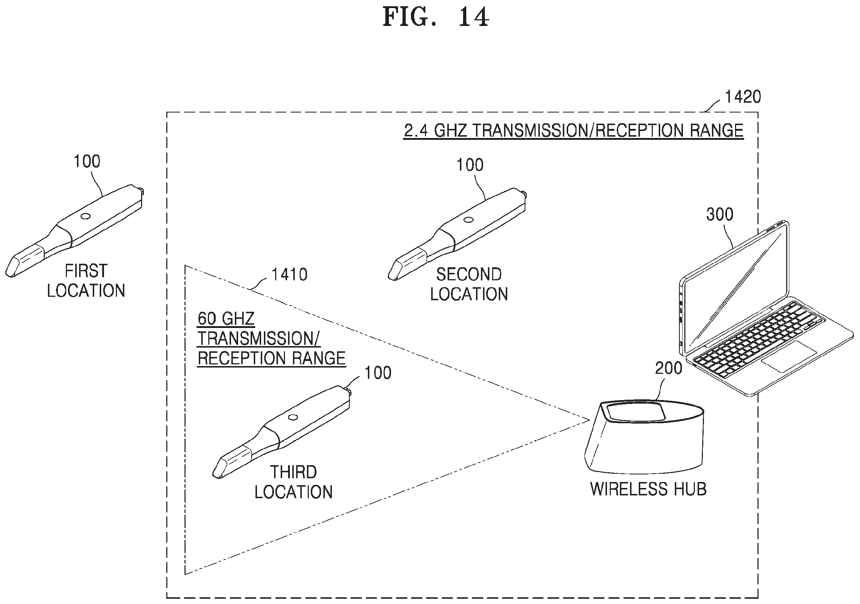

FIG. 14 is a reference diagram for explaining a signal transmission/reception range according to a location relationship between the wireless scanner 100 and the wireless hub 200 according to various examples.

Referring to FIG. 14, the location relationship between the wireless scanner 100, the wireless hub 200, and the data processing device 300 is shown.

A band frequency of 60 GHz used by the wireless scanner 100 and a second communication module 212 of the wireless hub 200 may have a very limited signal transmission/reception range 1410 due to the characteristics that an arrival distance of signal based on band frequency of 60 GHz is not long and the transmittance is low.

A band frequency of 2.4 GHz used by the wireless scanner 100 and a first communication module 211 of the wireless hub 200 may have, for example, a signal transmission/reception range 1420.

When the wireless scanner 100 is disposed at a first location, because the first location is out of both the 2.4 GHz signal transmission/reception range 1420 and the 60 GHz signal transmission/reception range 1410, transmission/reception of scan data and transmission/reception of control signals between the wireless scanner 100 and the wireless hub 200 are impossible.

When the wireless scanner 100 is disposed at a second location, the second location is within the 2.4 GHz signal transmission/reception range 1420, but is out of the 60 GHz signal transmission/reception range 1410, and thus, transmission/reception of control signals between the wireless scanner 100 and the wireless hub 200 is possible, but transmission/reception of scan data is impossible.

When the wireless scanner 100 is disposed at a third location, the third location is within the 2.4 GHz signal transmission/reception range 1420 and the 60 GHz signal transmission/reception range 1410, and thus, transmission/reception of scan data as well as transmission/reception of control signals between the wireless scanner 100 and the wireless hub 200 are possible.

A signal transmission/reception state between the wireless scanner 100 and the wireless hub 200 varies according to a location of the wireless scanner 100, and for smooth communication between the wireless scanner 100 and the wireless hub 200, the location of at least one of the wireless scanner 100 or the wireless hub 200 may be preferably adjusted. Therefore, according to the embodiments, the wireless hub 200 may operate to detect a communication state corresponding to signal transmission/reception between the wireless scanner 100 and the wireless hub 200 and provide feedback to a user in response to the detected communication state. The user feedback provided in response to the detected communication state may be provided by the data processing device 300 or by the wireless hub 200 by itself by allowing the wireless hub 200 to provide relevant information to the data processing device 300.

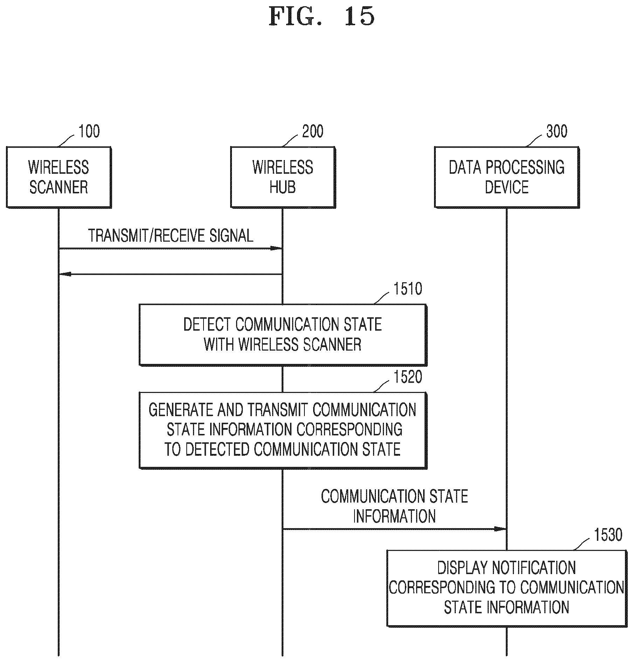

FIG. 15 is a flowchart illustrating an operation of a method of providing user feedback according to a communication state between the wireless scanner 100 and the wireless hub 200 according to an embodiment.

Referring to FIG. 15, in operation 1510, the wireless hub 200 may detect the communication state with the wireless scanner 100. The communication state between the wireless scanner 100 and the wireless hub 200 may include a plurality of states, as described above with reference to FIG. 14. A first communication state may indicate a state in which both a first communication module and a second communication module are not communicable because the wireless scanner 100 is out of a 2.4 GHz band range. A second communication state may indicate that the wireless scanner 100 is in the 2.4 GHz band range, but is out of a 60 GHz band range, that is, a state in which the first communication module is communicable but the second communication module is not communicable. A third communication state may indicate a state in which the wireless scanner 100 enters the 60 GHz band range and both the first communication module and the second communication module are communicable.

In operation 1520, the wireless hub 200 may generate communication state information corresponding to the detected communication state and transmit the generated communication state information to the data processing device 300.

According to an embodiment, the wireless hub 200 may generate the communication state information including one of the first communication state, the second communication state, and the third communication state according to the communication state detected in operation 1510.

In operation 1530, the data processing device 300 receiving the communication state information from the wireless hub 200 may display a notification corresponding to the received communication state information. Specifically, the data processing device 300 may display an appropriate notification corresponding to any one of the first communication state, the second communication state, and the third communication state included in the received communication state information.

According to an embodiment, the data processing device 300 may store a notification message to be displayed corresponding to each state that may be included in the communication state information received from the wireless hub 200. For example, the data processing device 300 may store a message indicating that the wireless scanner 100 is not connected, such as "not connected to scanner" or "scanner not found," in response to the first communication state. For example, the data processing device 300 may store a message guiding the wireless scanner 100 to be closer to the wireless hub 200 for scan data transmission/reception, such as "The wireless signal is not smooth" or "Please place the scanner closer to the hub," in response to the second communication state. The data processing device 300 may store a message indicating a state in which the data processing device 300 is communicable with the wireless scanner 100, for example, a message such as "a scan operation is normally performed" in a memory, in response to the third communication state. Therefore, the data processing device 300 may obtain a corresponding message according to a state included in the communication state information received from the wireless hub 200 and display the message on a display.

In the example shown in FIG. 15, the first communication state, the second communication state, and the third communication state have been described as the communication state information, but the embodiments are not limited thereto.

According to an embodiment, the communication state information may include only some of the first communication state, the second communication state, and the third communication state.

According to an embodiment, the communication state information may include an additional state in addition to the first communication state, the second communication state, and the third communication state. For example, the communication state information may further include at least one of a state in which the wireless hub 200 is being connected while searching for the wireless scanner 100, or a state in which the wireless scanner 100 is connected and disconnected.

According to an embodiment, the data processing device 100 may output the notification message by using various modalities. For example, the data processing device 100 may display the notification message by using a display pop-up window. For example, the data processing device 100 may provide a region on a part of a screen of the display that informs a communication state with the wireless scanner 100, and display the notification message on the region. For example, the data processing device 100 may convert the notification message into a voice and output the voice through an audio output unit.

FIG. 15 shows that the data processing device 300 receives and outputs the communication state information indicating the communication state between the wireless scanner 100 and the wireless hub 200, but embodiments are not limited thereto. According to embodiments, the wireless hub 200 may output the communication state between the wireless scanner 100 and the wireless hub 200 by itself.

According to an embodiment, the wireless hub 200 may output a notification corresponding to the communication state information indicating the communication state between the wireless scanner 100 and the wireless hub 200 by using a voice message.

According to an embodiment, the wireless hub 200 may output the notification corresponding to the communication state information indicating the communication state between the wireless scanner 100 and the wireless hub 200 by using lighting means such as an LED.

According to an embodiment, the wireless hub 200 may output the notification corresponding to the communication state information indicating the communication state between the wireless scanner 100 and the wireless hub 200 through vibration using a vibration sensor.

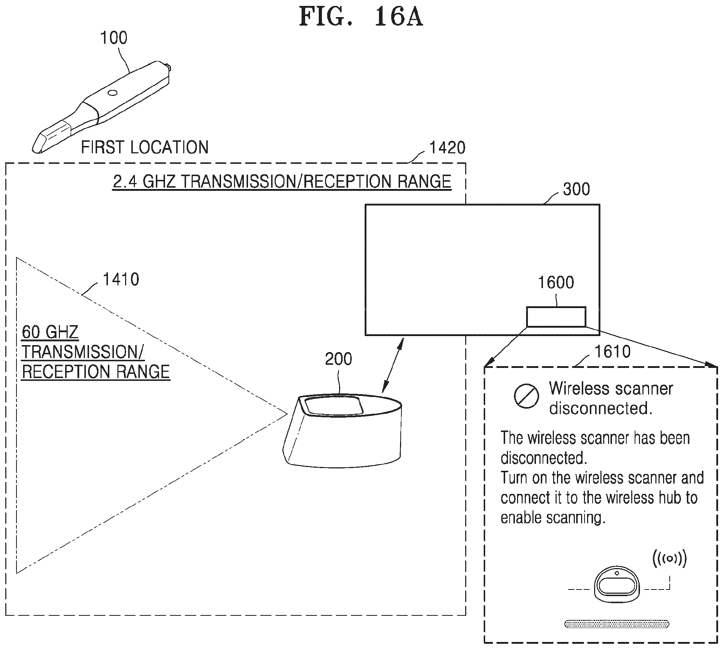

FIG. 16A is a reference diagram for explaining a notification operation of the data processing device 300 in a state in which both a first communication module and a second communication module are not communicable because the wireless scanner 100 is out of a 2.4 GHz band range according to an embodiment.

Referring to FIG. 16A, the wireless scanner 100 is located at a first location out of the 2.4 GHz band range. In this case, the wireless hub 200 may detect a first communication state in which the wireless scanner 100 is not communicable with both the first communication module and the second communication module, and transmit communication state information indicating that a wireless communication state with the wireless scanner 100 is in the first communication state to the data processing device 300. As the data processing device 300 receives the communication state information from the wireless hub 200 and confirms that the communication state information is in the first communication state, the data processing device 300 may display a message indicating that the wireless scanner 100 is not connected in response to the first communication state. For example, the data processing device 300 may display a message, such as "not connected to scanner" or "scanner not found," on a display as a notification corresponding to the first communication state.

Alternatively, as shown in FIG. 16A, the data processing device 300 may provide a region 1600 indicating the communication state between the wireless scanner 100 and the wireless hub 200 in a partial region of the display, and display a communication state such as "Wireless scanner disconnected" on the region 1600. The information about the wireless communication state between the wireless scanner 100 and the wireless hub 200 may be provided through the data processing device 300, thereby providing user feedback that enables a user to facilitate communication between the wireless scanner 100 and the wireless hub 200.

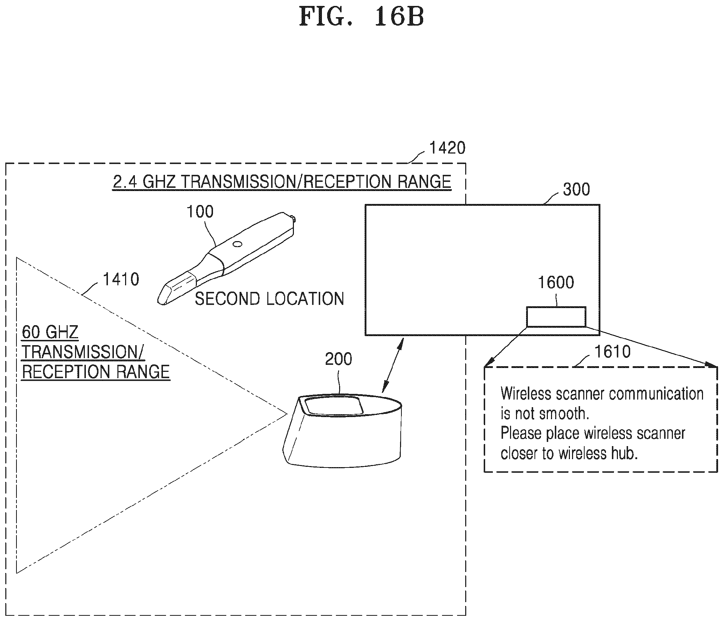

FIG. 16B is a reference diagram for explaining a notification operation of a data processing device in a state in which the wireless scanner 100 is in a 2.4 GHz band range but is out of a 60 GHz band range, that is, a first communication module is communicable but a second communication module is not communicable.

Referring to FIG. 16B, the wireless scanner 100 is located at a second location that is in the 2.4 GHz band range, but is out of the 60 GHz band range. In this case, the wireless hub 200 may detect a second communication state in which the wireless scanner 100 is communicable with the first communication module, but is not communicable with the second communication module, and transmit communication state information indicating that a wireless communication state with the wireless scanner 100 is in the second communication state to the data processing device 300. As the data processing device 300 receives the communication state information from the wireless hub 200 and confirms that the communication state information is in the second communication state, the data processing device 300 may display a message guiding the wireless scanner 100 to be closer to the wireless hub 200 for scan data transmission/reception, such as "The wireless signal is not smooth" or "Please place the scanner closer to the hub," in response to the second communication state. For example, as shown in FIG. 16B, the data processing device 300 may display a communication state, such as "Wireless scanner communication is not smooth. Please place wireless scanner closer to wireless hub.", on the region 1600 indicating the communication state between the wireless scanner 100 and the wireless hub 200 in a partial region of the display. The information about the wireless communication state between the wireless scanner 100 and the wireless hub 200 may be provided through the data processing device 300, thereby providing user feedback that enables a user to facilitate communication between the wireless scanner 100 and the wireless hub 200.

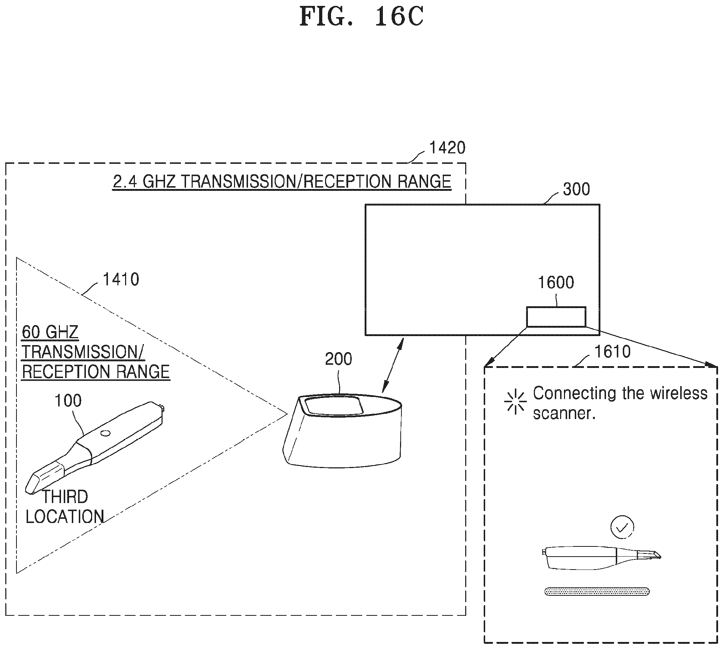

FIG. 16C is a reference diagram for explaining a notification operation of a data processing device in a state in which the wireless scanner 100 enters a 60 GHz band range and is communicable with both a first communication module and a second communication module according to an embodiment.

Referring to FIG. 16C, the wireless scanner 100 is located at a third location in the 60 GHz band range. In this case, the wireless hub 200 may detect a third communication state in which the wireless scanner 100 is communicable with both the first communication module and the second communication module, and transmit communication state information indicating that a wireless communication state with the wireless scanner 100 is in the third communication state to the data processing device 300. As the data processing device 300 receives the communication state information from the wireless hub 200 and confirms that the communication state information is in the third communication state, the data processing device 300 may display a message indicating a state in which the data processing device 300 is communicable with the wireless scanner 100, for example, a message such as "a scan operation is normally performed" in response to the third communication state. For example, as shown in FIG. 16C, the data processing device 300 may display a communication state, such as "Connecting the wireless scanner", on the region 1600 indicating the communication state between the wireless scanner 100 and the wireless hub 200 in a partial region of the display. The information about the wireless communication state between the wireless scanner 100 and the wireless hub 200 may be provided through the data processing device 300, thereby providing user feedback that enables a user to facilitate communication between the wireless scanner 100 and the wireless hub 200.

### Hub fan control according to scanner mode

Components included in the wireless hub 200 may include a plurality of circuit boards. For example, the transmission interface 220 in the wireless hub 200 may configured as a main printed circuit board (PCB), the first communication module 211 in the communication unit 210 may configured as a sub PCB, and the second communication module 212, the main PCB, and the sub PCB may each generate heat according to their operations. As described above, when each circuit component generates heat, the transmission/reception efficiency of scan data may decrease. Therefore, the wireless hub 200 may preferably eliminate heat generation of the components in the wireless hub 200 according to an operating state. As described above, the heat generation of the wireless hub 200 may be controlled, and thus, internal heat may be effectively dissipated to ensure reliable operations of the internal components of the wireless hub 200 as well as the authentication of a medical device may be satisfied due to the nature of the wireless hub 200 used as the medical device.

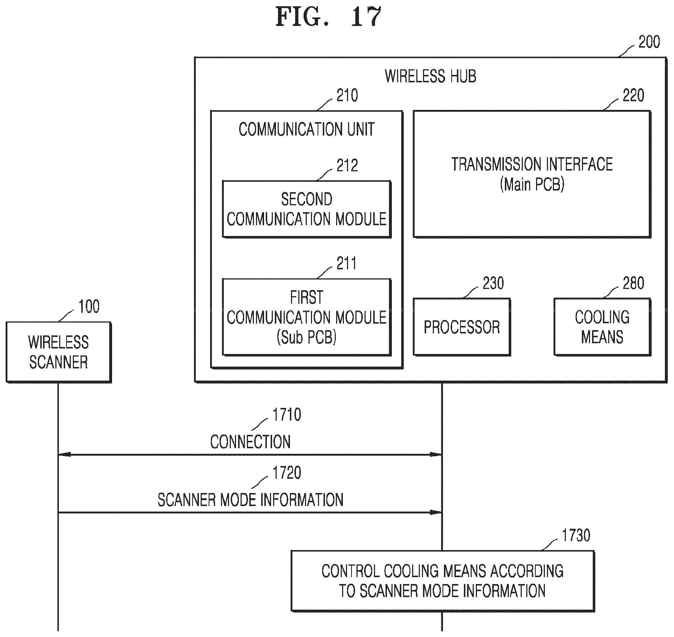

FIG. 17 is a flowchart illustrating an operation of a method of controlling heat generation in the wireless hub 200 according to an embodiment.

Referring to FIG. 17, the wireless hub 200 may further include cooling means 280 for cooling heat generated from various circuit components in the wireless hub 200. The cooling means 280 may include, for example, a fan.

In operation 1710, the wireless scanner 100 and the wireless hub 200 may perform a communication connection operation.

In operation 1720, the wireless hub 200 may receive scanner mode information from the wireless scanner 100. Specifically, the first communication module 211 of the wireless hub 200 may receive the scanner mode information from the wireless scanner 100 and transmit the received scanner mode information to the processor 230.

In operation 1730, the wireless hub 200 may control the cooling means 280 according to the received scanner mode information. Specifically, the processor 230 may control an operation of the cooling means 280 according to the received scanner mode information.

FIG. 18 is a reference diagram for describing scanner mode information according to an embodiment.

A scanner mode may have a plurality of mode states corresponding to various states of the wireless scanner 100.

Referring to FIG. 18, according to an embodiment, the scanner mode may include four states, a scan mode, an idle mode, a deep sleep mode, and an off mode.

The scan mode indicates a state in which the wireless scanner 100 is being scanned, and may indicate a state in which a camera, a projector, a fan, a first communication module, and a second communication module included in the wireless scanner 100 are all turned on.

The idle mode indicates a state in which the wireless scanner 100 is not currently scanning, but may immediately perform scanning when a scan button provided in the wireless scanner 100 is pressed. That is, when a scan stop button is pressed while scanning or the wireless scanner 100 is mounted on a cradle holding the wireless scanner 100 for a certain period of time or longer, the wireless scanner 100 may enter the idle mode. The state in which the wireless scanner 100 is mounted on the cradle for the certain period of time or longer may be detected, for example, by a gyro sensor provided in the wireless scanner 100. In such an idle mode, the camera of the wireless scanner 100 may be in an on or off state, the projector may be in the off state, and both the first communication module and the second communication module may be in the on state. In the idle mode, the fan may be in an automatically controlled state according to the temperature of the wireless scanner 100.

The deep sleep mode may be entered when a certain period of time elapses after the wireless scanner 100 enters the sleep mode, that is, after entering the idle mode. In such a deep sleep mode, when the scan button of the wireless scanner 100 is pressed, it may take a certain period of time, for example, about 3 seconds to about 5 seconds, to connect the second communication module. In such a deep sleep mode, the camera of the wireless scanner 100 may be in the off state, the projector may be in the off state, and the second communication module may be in the off state. In addition, the first communication module may be in the on state, and the fan may be in the automatically controlled state according to the temperature of the wireless scanner 100.

The off mode refers to a state in which the power of the wireless scanner 100 is turned off, and in order to operate the wireless scanner 100, a power button needs to be pressed. In the off mode, the camera, the projector, the fan, the first communication module, and the second communication module included in the wireless scanner 100 may all be in the off state.

Referring back to FIG. 17, in operation 1730, the wireless hub 200 may control the cooling means 280 according to, for example, the scanner mode information as shown in FIG. 17. Specifically, when the received scanner mode information indicates the scan mode, the processor 230 may control cooling means 280 to operate because the second communication module, the main PCB, and the sub PCB are all in the on state for transmission and reception operations of scan data and a control signal. When the received scanner mode information indicates any one of the idle mode, the deep sleep mode, or the off mode, the processor 230 does not require an operation of transmitting/receiving the scan data and needs to turn on only the sub PCB for transmitting/receiving the control signal, and the processor 230 may control the cooling means 280 not to operate. The sub PCB does not consume much power and thus does not generate much heat, when only the sub PCB operates, a problem due to heat generation may not occur even when the cooling means 280 does not operate.

In the example shown in FIG. 17, it has been described that the scanner mode information is received from the wireless scanner 100 and the cooling means 280 is controlled according to the received scanner mode information, but the embodiments are not limited thereto.

According to an embodiment, the wireless hub 200 may control the operation of the cooling means 280 according to the temperature of the wireless hub 200 due to heat generation of the wireless hub 200 according to the on and off states of the second communication module, the main PCB, and the sub PCB. Specifically, the wireless hub 200 may store temperature information of the wireless hub 200 due to the heat generation of the wireless hub 200 according to the on and off states of the second communication module, the main PCB, and the sub PCB, and control an operating speed or an operating time of the cooling means 280 according to the temperature information. For example, the wireless hub 200 may store temperature information of the wireless hub 200 due to the heat generation of the wireless hub 200 according to the on and off states of the second communication module, the main PCB, and the sub PCB as a first value, a second value, and a third value, and store the operating speed of the cooling means 280 as a first speed in response to the temperature information first value, the operating speed of the cooling means 280 as a second speed in response to the temperature information second value, and the operating speed of the cooling means 280 as a third speed in response to the temperature information third value. As described above, the wireless hub 200 may adaptively control the cooling means 280 by varying the operating speed of the cooling means 280 according to the temperature of the wireless hub 200.

According to an embodiment, the wireless hub 200 may arrange a noise sensor outside the wireless hub 200 and control the operation of the cooling means 280 according to a value measured by the noise sensor. For example, the wireless hub 200 may determine that noise is low based on the value measured by the noise sensor and lower the operating speed of the cooling means 280, thereby preventing a user from being affected by the noise caused by the cooling means 280 as much as possible.

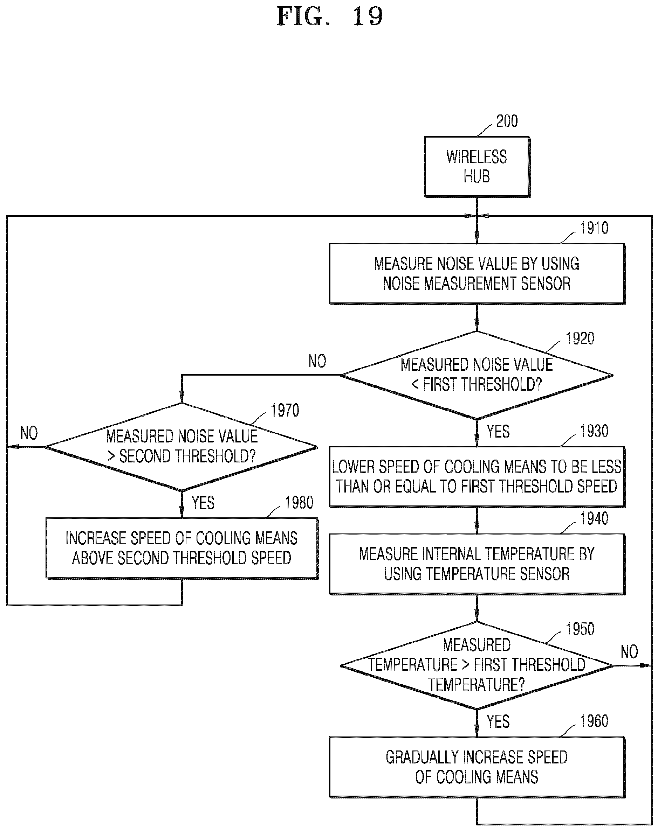

FIG. 19 is a flowchart illustrating an example of an operation method of the wireless hub 200 according to a noise measurement value according to an embodiment.

Referring to FIG. 19, in operation 1910, the wireless hub 200 may measure a noise value by using a noise measurement sensor. The wireless hub 200 may include the noise measurement sensor that measures the magnitude of noise by converting sound received through a microphone into an electrical signal and measuring the magnitude thereof.

In operation 1920, the wireless hub 200 may determine whether the measured noise value is less than a first threshold. As a result of the determination, when the measured noise value is not less than the first threshold, that is, when the noise value is not very low, the wireless hub 200 may proceed to operation 1910 and continue to measure the noise value. As a result of the determination, when the measured noise value is less than the first threshold, that is, when it is determined that the noise value is very low, the wireless hub 200 may proceed to operation 1930.

In operation 1930, the wireless hub 200 may lower a speed of cooling means to be less than or equal to a first threshold speed. That is, when the noise value is less than the first threshold, the wireless hub 200 may lower the speed of the cooling means to be less than or equal to the first threshold speed, thereby reducing noise caused by the operation of the cooling means. This is to eliminate user's inconvenience by reducing the noise of the cooling means because the noise caused by the operation of the cooling means of the wireless hub 200 may be sensitive to the user when an external environment is quiet.

In operation 1940, the wireless hub 200 may measure the internal temperature by using a temperature sensor. The wireless hub 200 includes the temperature sensor that measures the temperature in the wireless hub 200, and may measure the internal temperature of the wireless hub 200 by using the temperature sensor.

In operation 1950, the wireless hub 200 may determine whether the internal temperature measured by using the temperature sensor is higher than a first threshold temperature. Because the speed of the cooling means is lowered below the first threshold speed in operation 1930, the internal temperature of the wireless hub 200 may increase when such a state is maintained. However, because it is not continuously allowed to increase the internal temperature of the wireless hub 200 for reduction of noise, the wireless hub 200 may determine whether the internal temperature is higher than the first threshold temperature so as to prevent the internal temperature from increasing above a certain level.

As a result of the determination, when the internal temperature of the wireless hub 200 is not higher than the first threshold temperature, the wireless hub 200 may proceed to operation 1910. As a result of the determination, when the internal temperature of the wireless hub 200 is higher than the first threshold temperature, the wireless hub 200 may proceed to operation 1960.

In operation 1960, the wireless hub 200 may gradually increase the speed of the cooling means. That is, when the internal temperature of the wireless hub 200 is higher than the first threshold temperature, the wireless hub 200 may gradually increase the speed of the cooling means so as to gradually decrease the internal temperature of the wireless hub 200. In addition, the wireless hub 200 used together with a wireless scanner scanning an oral cavity is one of medical devices, and may have temperature requirements necessary for authentication of medical devices. Therefore, when the internal temperature of the wireless hub 200 exceeds the temperature according to the temperature requirement while reducing the speed of the cooling means due to a very low noise environment, to compensate for this, temperature conditions are prioritized, and an increase in the speed of the cooling means may be necessary.

When the measured noise value is not less than the first threshold as a result of the determination in operation 1920, that is, when the external environment is noisy to some extent, the wireless hub 200 may proceed to operation 1970.

In operation 1970, the wireless hub 200 may determine whether the measured noise value is greater than a second threshold value. When the measured noise value is not greater than the second threshold value as a result of the determination, the wireless hub 200 may proceed to operation 1910. When the measured noise value is greater than the second threshold value as a result of the determination, the wireless hub 200 may proceed to operation 1980.

In operation 1980, the wireless hub 200 may increase the speed of the cooling means above a second threshold speed. That is, when the wireless hub 200 determines that the measured noise value is greater than the second threshold and is a noisy environment to an extent, the noise caused by the operation of the cooling means may not incur much inconvenience to the user, the wireless hub 200 may focus more on lowering the internal temperature of the wireless hub 200 by increasing the speed of the cooling means.

### Display of scanner state on display device based on scanner mode

The wireless scanner 100 may provide information about a state of the wireless scanner 100 itself to the wireless hub 200 periodically or when the state changes. The information about the state of the wireless scanner 100 may be, for example, scanner mode information as shown in FIG. 18.

In addition to controlling the cooling means as described above with reference to FIG. 15 by using the received scanner mode information when receiving the scanner mode information from the wireless scanner 100, the wireless hub 200 may transmit the scanner mode information to the data processing device 300 to output the information about the state of the wireless scanner 100, thereby allowing the user to recognize the information about the state of the wireless scanner 100.

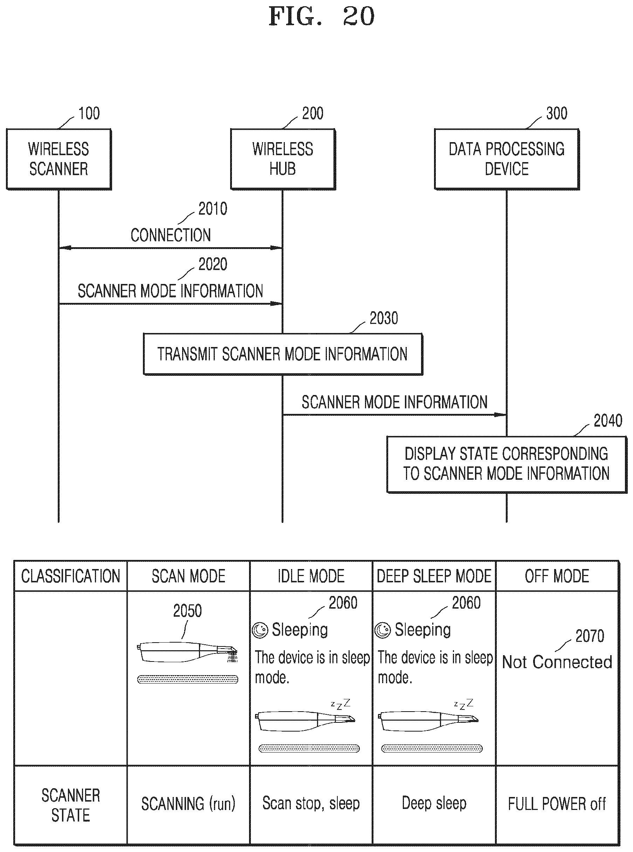

FIG. 20 is a flowchart illustrating an operation of a method of displaying state information of the wireless scanner 100 according to an embodiment.

Referring to FIG. 20, in operation 2010, the wireless scanner 100 and the wireless hub 200 may perform a communication connection operation.

In operation 2020, the wireless hub 200 may receive scanner mode information from the wireless scanner 100. Specifically, the first communication module 211 of the wireless hub 200 may receive the scanner mode information from the wireless scanner 100 and transmit the received scanner mode information to the processor 230. The wireless scanner 100 may periodically transmit the scanner mode information to the wireless hub 200, transmit the scanner mode information to the wireless hub 200 when a specific event occurs, transmit the scanner mode information at a request of the wireless hub 200, or transmit the scanner mode information to the wireless hub 200 whenever a scanner mode of the wireless scanner 100 is changed.

In operation 2030, the wireless hub 200 may transmit the received scanner mode information to the data processing device 300.

In operation 2040, the data processing device 300 may receive the scanner mode information of the wireless scanner 100 from the wireless hub 200 and display a state corresponding to the received scanner mode information.

Specifically, when the scanner mode information indicates that the wireless scanner 100 is in a scan mode, the data processing device 300 may display an image 2050 corresponding to the scan mode on at least a partial region of a display to indicate that the wireless scanner 100 is scanning.

When the scanner mode information indicates that the wireless scanner 100 is in an idle mode or a deep sleep mode, the data processing device 300 may display an image 2060 on at least a partial region of the display to indicate that the wireless scanner 100 is waiting to scan.

When the scanner mode information indicates that the wireless scanner 100 is in an off mode, the data processing device 300 may display an image 2070 on at least a partial region of the display to indicate that the wireless scanner 100 is in an off state.

As described above, an operating state of the wireless scanner 100 may be displayed through the data processing device 300, thereby allowing a user to easily know the operating state of the wireless scanner 100.

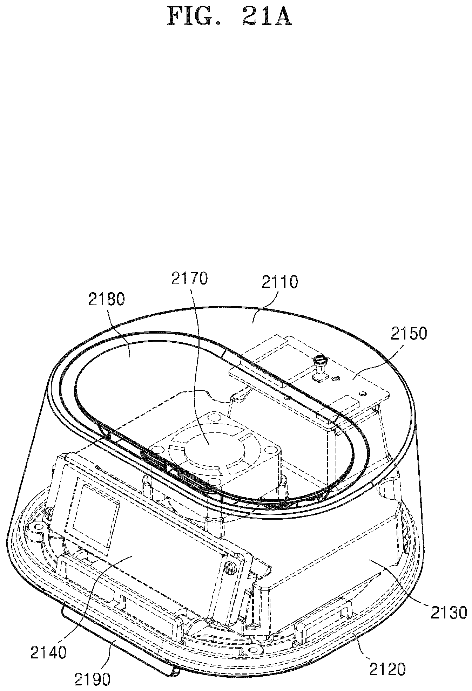

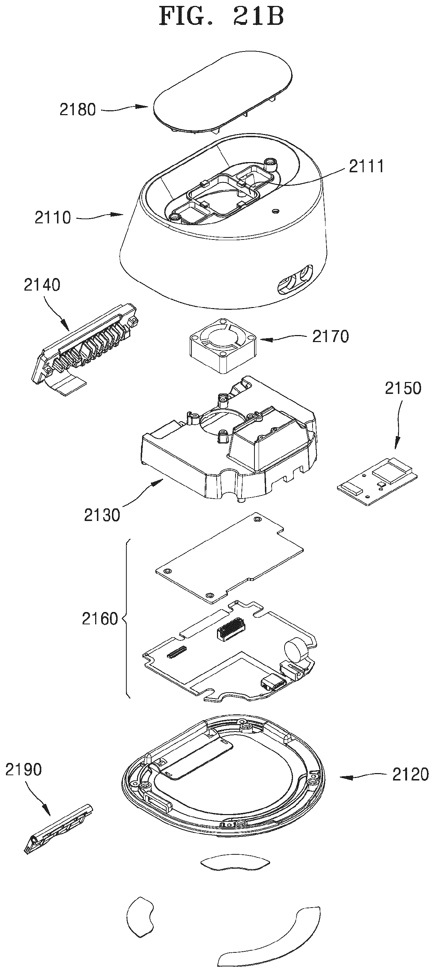

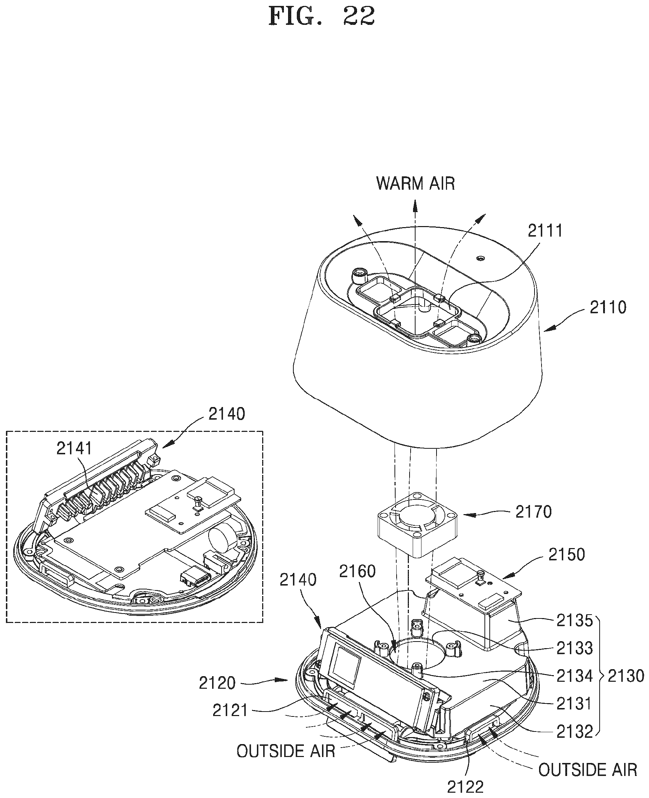

When the wireless hub 200 operates to receive a scanning signal from the wireless scanner 100, circuit components in the wireless hub 200 generate a lot of heat, and the temperature inside a case of the wireless hub 200 increases, which may reduce transmission/reception efficiency of image and a control signal. Therefore, it is important to minimize the heat generated by the operation of the wireless hub 200. Therefore, according to various embodiments of the present disclosure, the wireless hub 200 may include means to dissipate heat generated by each communication module and means to discharge hot air generated within the wireless hub 200. Hereinafter, embodiments of the wireless hub 200 having a structure capable of eliminating heat generation of the wireless hub 200 are described with reference to FIGS. 21A, 21B, and 22.

FIG. 21A illustrates an example of a structure of the wireless hub 200 for eliminating heat generation of components according to an embodiment.

FIG. 21B illustrates an example of an exploded view of the wireless hub 200 according to an embodiment.

Referring to FIGS. 21A and 21B, the wireless hub 200 may include an upper case 2110 and a lower case 2120 for surrounding internal components.

The upper case 2110 may include an air discharge hole 2111 for discharging air discharged from cooling means 2170 disposed therebelow to the outside of the wireless hub 200.

The upper case 2110 may be coupled to an upper case cover 2180 for covering the air discharge hole 2111 of the upper case 2110. The upper case cover 2180 may prevent hot air discharged from the air discharge hole 2111 from being directly exposed to an upper end of the wireless hub 200, thereby preventing users near the wireless hub 200 from being exposed to burn.

An inner space of the wireless hub 200 surrounded by the upper case 2110 and the lower case 2120 may include a support 2130 for supporting internal components.

A main PCB 2160 may include the processor 230 shown in FIG. 2, and may be installed between the lower case 2120 and the support 2130.

A hinge 2190 capable of adjusting an angle may be disposed below the lower case 2120 so that one side of the wireless hub 200 may be inclined, thereby adjusting the angle of an antenna of a wireless communication module 2140.

The wireless communication module 2140, a sub PCB 2150, and the cooling means 2170 may be installed on the support 2130. The support 2130 may include a material for absorbing heat from the wireless communication module 2140, the main PCB 2160, and the sub PCB 2150. For example, the support 2130 may include a metal frame, for example, aluminum.

The wireless communication module 2140 may be disposed on one side of the support 2130, and may be tilted and disposed to optimize signal transmission/reception with the wireless scanner 100. A heat sink for absorbing heat generated by an operation of a circuit of the wireless communication module 2140 and dissipating the heat to the outside may be disposed in the wireless communication module 2140.

The wireless communication module 2140 may include the second communication module 212 shown in FIG. 2.

The sub PCB 2150 may be disposed on a structure 2135 provided on the support 2130 so as to be spaced apart from the wireless communication module 2140 or the main PCB 2160 disposed below the support 2130. The sub PCB 2150 may include the first communication module 211 shown in FIG. 2.

The cooling means 2170 may be disposed above a hole in the middle of the support 2130 to operate to discharge heat generated from the wireless communication module 2140 installed on one side of the support 2130, heat generated from the sub PCB 2150 installed on the other side of the support 2130, and heat generated from the main PCB 2160 disposed below the support 2130 to the outside of the wireless hub 200. The air discharged from the cooling means 2170 may be discharged to the outside of the wireless hub 200 through the air discharge hole 2111 of the upper case 2110. The cooling means 2170 may include, for example, a fan.

FIG. 22 is a reference diagram for describing a structure of the wireless hub 200 for eliminating heat generation of components according to an embodiment.

Referring to FIG. 22, the support 2130 may include a support plate 2131 and a sidewall 2132 provided below the support plate 2131 along the circumference of the support plate 2131. The sidewall 2132 of the support 2130 may be seated on the lower case 2120. The main PCB 2160 may be accommodated in a space surrounded by the sidewall 2132 below the support plate 2131.

A hole 2133 may be provided in the center of the support plate 2131, and a plurality of fastening units 2134 for fastening the cooling means 2170 may be provided around the hole 2133. The cooling means 2170 may be fastened to the fastening units 2134 and disposed in an upper portion of the hole 2133. Heat generated from the main PCB 2160 may be transferred to the cooling means 2170 through the hole 2133.

The wireless communication module 2140 may be disposed at one side of the support plate 2131 with respect to the hole 2133. In this regard, the first communication module 2140 may be tilted and disposed to increase signal transmission/reception efficiency with the wireless scanner 100.

The structure 2135 in which the sub PCB 2150 may be located may be disposed on the other side of the support plate 2131 with respect to the hole 2133. The sub PCB 2150 may be installed on the structure 2135 so that the sub PCB 2150 may be spaced apart from the main PCB 2160 disposed below the support plate 2131. As described above, the sub PCB 2150 may be spaced apart from the main PCB 2160, thereby reducing influence of heat generated between the sub PCB 2150 and the main PCB 2160 on each other.

Heat generated from the wireless communication module 2140 disposed on one side of the support plate 2131 with respect to the hole 2133, heat generated from the sub PCB 2150 arranged on the other side of the support plate 2131 with respect to the hole 2133, and heat generated from the main PCB 2160 disposed below the support plate 2131 may be discharged to the outside of the wireless hub 200 through the air discharge hole 2111 via the cooling means 2170 disposed in the upper portion of the hole 2133.

In addition, in order to more effectively discharge heat generated by the wireless communication module 2140, the main PCB 2160, and the sub PCB 2150 inside the wireless hub 200, means to ventilate cold air from the outside of the wireless hub 200 is required. To this end, one or more ventilation holes 2121 and 2122 may be provided in the outer periphery of the lower case 2120. For example, one, two, three, or more air ventilation holes may be provided in the lower case 2120.

As described above, the wireless communication module 2140, the sub PCB 2150, and the main PCB 2160 may be disposed on the support plate 2131, and hot air may be discharged to the upper end of the wireless hub 200 through cooling means 2170, and thus, the heat generated by such circuit components may be efficiently reduced, thereby optimizing operations of such circuit components.

In addition, a heat sink 2141 for absorbing heat generated by the operation of the circuit of the wireless communication module 2140 and dissipating the heat to the outside may be disposed in the wireless communication module 2140.

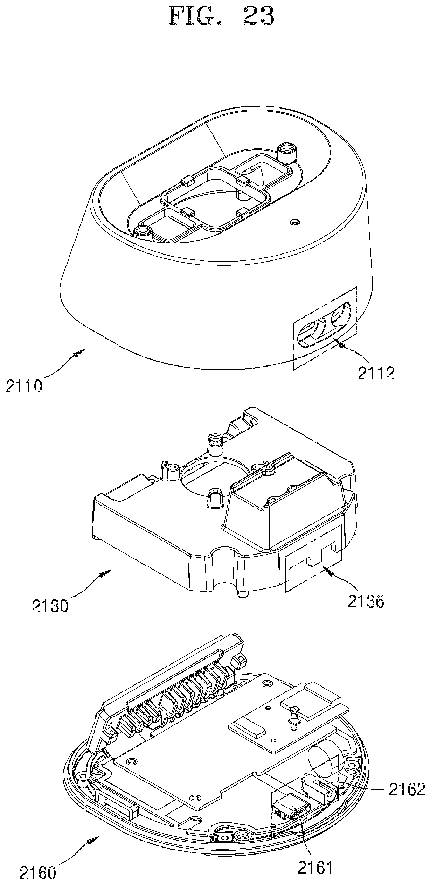

FIG. 23 is a reference diagram for describing a connection structure for the wireless hub 200 to communicate with the data processing device 300 according to an embodiment.

Referring to FIG. 23, the wireless hub 200 may include a transmission/reception interface 2161 and a power interface 2162 on the main PCB 2160 of the wireless hub 200 to transmit scan data received from the wireless scanner 100 to the data processing device 300.

A cable may be connected to the transmission/reception interface 2161, and data from the wireless hub 200 may be transmitted to the data processing device 300 through the cable. The data transmitted from the wireless hub 200 to the data processing device 300 may include scan data, a control signal, and state information or mode information of the wireless hub 200 or the wireless scanner 100.

A power cable may be connected to the power interface 2162 to receive power.

In the example shown in FIG. 23, the power interface 2162 is provided separately from the data transmission/reception interface 2161, but the wireless hub 200 may also receive power from the data processing device 300 through the transmission/reception interface 2161. The transmission/reception interface 2161 may be implemented to not only transmit the data from the wireless hub 200 to the data processing device 300 through the connected cable, but also receive power from the data processing device 300.

An opening 2136 may be provided in the support 2130 and an opening 2112 may be provided in the upper case 2110 to pass the cable connected to the transmission/reception interface 2161 and the power interface 2162.

An operation method of an electronic device according to an embodiment of the present disclosure may be recorded on a computer-readable recording medium by being implemented in a form of program commands executed by using various computers. Also, an embodiment of the present disclosure may include a computer-readable storage medium having recorded thereon at least one program including at least one instruction for executing the operation method of the electronic device.

The computer-readable recording medium may include at least one of a program command, a data file, or a data structure. Examples of the computer-readable recording medium include magnetic media such as hard disks, floppy disks, and magnetic tapes, optical media such as CD-ROMs and DVDs, magneto-optical media such as floptical disks, and hardware devices configured to store and perform program commands, such as ROM, RAM, and flash memory.

A machine-readable storage medium may be provided in the form of a non-transitory storage medium. The "non-transitory storage medium" may denote that a storage medium is a tangible device. The "non-transitory storage medium" may include a buffer where data is temporarily stored.

According to an embodiment, the operation method of the electronic device according to various embodiments in the present specification may be provided by being included in a computer program product. The computer program product may be distributed in the form of the machine-readable storage medium (e.g., a CD-ROM). Alternatively, the computer program product may be distributed (e.g., downloaded or uploaded) directly or online through an application store (e.g., PlayStore^{™}) or between two user devices (e.g., smartphones). In detail, the computer program product according to an embodiment may include a storage medium having recorded thereon a program including at least one instruction for executing the operation method of the electronic device according to an embodiment.

While the embodiments have been particularly shown and described in detail, it will be understood by one of ordinary skill in the art that various changes in form and details may be made therein without departing from the scope of the present disclosure as defined by the following claims.

## Claims

1. An electronic device which receives scan data from an intraoral scanner (100) and transmits the scan data to a data processing device (300) processing the scan data, the electronic device (200) comprising:
a wireless communication module (210);
a memory comprising one or more instructions; and
a processor (230) configured to execute the one or more instructions to control the wireless communication module to receive the scan data transmitted from the intraoral scanner, and control the received scan data to be processed and transmitted to the data processing device,
wherein the wireless communication module comprises an antenna (214) for receiving the scan data transmitted from the intraoral scanner,
wherein the antenna is disposed or controlled to have at least one of a location or a direction suitable for receiving the scan data, and
wherein the processor is further configured to execute the one or more instructions to:
detect a communication state with the intraoral scanner through the wireless communication module of the electronic device, and
mechanically control at least one of:
an inclination direction of the antenna, according to the detected communication state, using a motorised tilt support (244) the inclination of which is controlled by a signal strength,
a rotation of the antenna, according to the detected communication state, using a motorised rotation support (243) the rotation of which is controlled by the signal strength,
an inclination direction of a housing of the electronic device where the antenna is installed, according to the detected communication state, using a motorised hinge (800) the inclination of which is controlled by the signal strength, or
a rotation of the housing of the electronic device where the antenna is installed, according to the detected communication state, using a motorised rotation plate (270) the rotation of which is controlled by the signal strength.

2. The electronic device of claim 1, wherein the housing of the electronic device includes the motorised hinge (800) for controlling an inclination of the antenna (214).

3. The electronic device of claim 1, wherein:
the antenna (214) is disposed to have an inclination on a side of the housing of the electronic device, and
the side of the housing of the electronic device is installed to have an inclination corresponding to the inclination of the antenna.

4. The electronic device of claim 1, wherein:
the wireless communication module (210) includes a second communication module (212) operating in a 60 GHz signal transmission/reception band, and
the processor (230) is configured to execute the one or more instructions to:
obtain at least one of the inclination direction of the antenna (214) or the inclination direction of the housing of the electronic device where the antenna is installed for which a strength of signal received from the second communication module exceeds a threshold, and
mechanically control at least one of the antenna or the housing of the electronic device according to at least one of the obtained inclination direction of the antenna or the obtained inclination direction of the housing.

5. The electronic device of claim 1, wherein the processor (230) is configured to execute the one or more instructions to:
detect a communication state with the intraoral scanner (100); and
provide user feedback guiding to adjust a location or a direction of at least one of the intraoral scanner or the electronic device in response to the detected communication state.

6. The electronic device of claim 5, wherein the processor (230) is configured to execute the one or more instructions to provide the user feedback guiding to adjust the location or the direction of at least one of the intraoral scanner (100) or the electronic device when the detected communication state indicates that communication is possible in a 2.4 GHz range and that communication is impossible in a 60 GHz range.

7. The electronic device of claim 6, wherein the processor (230) is configured to execute the one or more instructions to :
detect an operating state of the intraoral scanner (100), and
control cooling means of the electronic device according to the detected operating state of the intraoral scanner.

8. The electronic device of claim 5, wherein the processor (230) is configured to execute the one or more instructions to:
control cooling means of the electronic device to operate when an operating state of the intraoral scanner (100) indicates that the intraoral scanner is operating, and
control the cooling means of the electronic device not to operate when the operating state of the intraoral scanner indicates that the intraoral scanner is waiting to operate.

9. The electronic device of claim 7, wherein the processor (230) is configured to execute the one or more instructions to detect the operating state of the intraoral scanner (100) by receiving information about the operating state of the intraoral scanner from the intraoral scanner.

10. An operation method of an electronic device according to claim 1 which receives scan data from an intraoral scanner (100) and transmits the scan data a data processing device (300) processing scan data obtained by the intraoral scanner, the operation method comprising:
detecting a communication state with the intraoral scanner through a wireless communication module (210) of the electronic device, and
mechanically controlling at least one of:
an inclination direction of an antenna (214), according to the detected communication state, using a motorised tilt support (244) the inclination of which is controlled by a signal strength,
a rotation of the antenna, according to the detected communication state, using a motorised rotation support (243) the rotation of which is controlled by the signal strength,
an inclination direction of a housing of the electronic device where the antenna is installed, according to the detected communication state, using a motorised hinge (800) the inclination of which is controlled by the signal strength, or
a rotation of the housing of the electronic device where the antenna is installed, according to the detected communication state, using a motorised rotation plate (270) the rotation of which is controlled by the signal strength.

11. The operation method of claim 10, wherein the wireless communication module (210) of the electronic device includes a second communication module (212) operating in a 60 GHz signal transmission/reception band,
wherein the operation method further comprises:
obtaining at least one of the inclination direction of the antenna (214) or the inclination direction of the housing of the electronic device where the antenna is installed for which a strength of signal received from the second communication module exceeds a threshold, and
mechanically controlling at least one of the antenna or the housing of the electronic device according to at least one of the obtained inclination direction of the antenna or the obtained inclination direction of the housing.

12. The operation method of claim 10, further comprising:
detecting a communication state with the intraoral scanner (100); and
providing user feedback guiding to adjust a location or a direction of at least one of the intraoral scanner or the electronic device in response to the detected communication state.

13. The operation method of claim 12, further comprising: providing the user feedback guiding to adjust the location or the direction of at least one of the intraoral scanner (100) or the electronic device when the detected communication state indicates that communication is possible in a 2.4 GHz range and that communication is impossible in a 60 GHz range.

14. The operation method of claim 13, wherein the electronic device further includes cooling means to eliminate heat generation of the electronic device,
wherein the operation method further comprises:
detecting an operating state of the intraoral scanner (100), and
controlling the cooling means of the electronic device according to the detected operating state of the intraoral scanner.

## Patentansprüche

1. Elektronische Vorrichtung, die Scandaten von einem intraoralen Scanner (100) empfängt und die Scandaten an eine Datenverarbeitungsvorrichtung (300) überträgt, die die Scandaten verarbeitet, wobei die elektronische Vorrichtung (200) Folgendes umfasst:
ein drahtloses Kommunikationsmodul (210);
einen Speicher, der eine oder mehrere Anweisungen umfasst; und
einen Prozessor (230), der dazu konfiguriert ist, die eine oder die mehreren Anweisungen auszuführen, um das drahtlose Kommunikationsmodul so zu steuern, dass es die von dem intraoralen Scanner übertragenen Scandaten empfängt, und die empfangenen, zu verarbeitenden und an die Datenverarbeitungsvorrichtung zu übertragenden Scandaten zu steuern,
wobei das drahtlose Kommunikationsmodul eine Antenne (214) zum Empfangen der von dem intraoralen Scanner übertragenen Scandaten umfasst,
wobei die Antenne so angeordnet oder gesteuert ist, dass sie mindestens eine von einer Position oder einer Richtung aufweist, die zum Empfangen der Scandaten geeignet sind, und
wobei der Prozessor ferner dazu konfiguriert ist, die eine oder die mehreren Anweisungen zu Folgendem auszuführen:
Erfassen eines Kommunikationszustands mit dem intraoralen Scanner über das drahtlose Kommunikationsmodul der elektronischen Vorrichtung, und
mechanisches Steuern von mindestens einem von Folgendem:
einer Neigungsrichtung der Antenne, gemäß dem erfassten Kommunikationszustand, unter Verwendung einer motorisierten Neigungsstütze (244), deren Neigung durch eine Signalstärke gesteuert wird,
einer Drehung der Antenne, gemäß dem erfassten Kommunikationszustand, unter Verwendung einer motorisierten Drehstütze (243), deren Drehung durch die Signalstärke gesteuert wird,
einer Neigungsrichtung eines Gehäuses der elektronischen Vorrichtung, in der die Antenne installiert ist, gemäß dem erfassten Kommunikationszustand, unter Verwendung eines motorisierten Scharniers (800), dessen Neigung durch die Signalstärke gesteuert wird, oder
einer Drehung des Gehäuses der elektronischen Vorrichtung, in der die Antenne installiert ist, gemäß dem erfassten Kommunikationszustand, unter Verwendung einer motorisierten Drehplatte (270), deren Drehung durch die Signalstärke gesteuert wird.

2. Elektronische Vorrichtung nach Anspruch 1, wobei das Gehäuse der elektronischen Vorrichtung das motorisierte Scharnier (800) zum Steuern einer Neigung der Antenne (214) beinhaltet.

3. Elektronische Vorrichtung nach Anspruch 1, wobei:
die Antenne (214) so angeordnet ist, dass sie eine Neigung an einer Seite des Gehäuses der elektronischen Vorrichtung aufweist, und
die Seite des Gehäuses der elektronischen Vorrichtung so installiert ist, dass sie eine Neigung aufweist, die der Neigung der Antenne entspricht.

4. Elektronische Vorrichtung nach Anspruch 1, wobei:
das drahtlose Kommunikationsmodul (210) ein zweites Kommunikationsmodul (212) beinhaltet, das in einem 60-GHz-Signalsende-/-empfangsband in Betrieb ist, und
der Prozessor (230) dazu konfiguriert ist, die eine oder die mehreren Anweisungen zu Folgendem auszuführen:
Erhalten mindestens einer von der Neigungsrichtung der Antenne (214) oder der Neigungsrichtung des Gehäuses der elektronischen Vorrichtung, in der die Antenne installiert ist, für die eine Signalstärke, die von dem zweiten Kommunikationsmodul empfangen wird, einen Schwellenwert überschreitet, und
mechanisches Steuern mindestens eines von der Antenne oder dem Gehäuse der elektronischen Vorrichtung gemäß mindestens einer von der erhaltenen Neigungsrichtung der Antenne oder der erhaltenen Neigungsrichtung des Gehäuses.

5. Elektronische Vorrichtung nach Anspruch 1, wobei der Prozessor (230) dazu konfiguriert ist, die eine oder die mehreren Anweisungen zu Folgendem auszuführen:
Erfassen eines Kommunikationszustands mit dem intraoralen Scanner (100); und Bereitstellen einer Benutzerrückkopplungsführung, um eine Position oder eine Richtung von mindestens einem von dem intraoralen Scanner oder der elektronischen Vorrichtung als Reaktion auf den erfassten Kommunikationszustand einzustellen.

6. Elektronische Vorrichtung nach Anspruch 5, wobei der Prozessor (230) dazu konfiguriert ist, die eine oder die mehreren Anweisungen auszuführen, um die Benutzerrückkopplungsführung bereitzustellen, um die Position oder die Richtung von mindestens einem von dem intraoralen Scanner (100) oder der elektronischen Vorrichtung einzustellen, wenn der erfasste Kommunikationszustand angibt, dass eine Kommunikation in einem 2,4-GHz-Bereich möglich ist und dass eine Kommunikation in einem 60-GHz-Bereich unmöglich ist.

7. Elektronische Vorrichtung nach Anspruch 6, wobei der Prozessor (230) dazu konfiguriert ist, die eine oder die mehreren Anweisungen zu Folgendem auszuführen:
Erfassen eines Betriebszustands des intraoralen Scanners (100) und Steuern einer Kühleinrichtung der elektronischen Vorrichtung gemäß dem erfassten Betriebszustand des intraoralen Scanners.

8. Elektronische Vorrichtung nach Anspruch 5, wobei der Prozessor (230) dazu konfiguriert ist, die eine oder die mehreren Anweisungen zu Folgendem auszuführen:
Steuern einer Kühleinrichtung der elektronischen Vorrichtung derart, dass sie in Betrieb ist, wenn ein Betriebszustand des intraoralen Scanners (100) angibt, dass der intraorale Scanner in Betrieb ist, und
Steuern der Kühleinrichtung der elektronischen Vorrichtung derart, dass sie nicht in Betrieb ist, wenn der Betriebszustand des intraoralen Scanners angibt, dass der intraorale Scanner darauf wartet, in Betrieb zu sein.

9. Elektronische Vorrichtung nach Anspruch 7, wobei der Prozessor (230) dazu konfiguriert ist, die eine oder die mehreren Anweisungen auszuführen, um den Betriebszustand des intraoralen Scanners (100) durch Empfangen von Informationen über den Betriebszustand des intraoralen Scanners von dem intraoralen Scanner zu erfassen.

10. Betriebsverfahren einer elektronischen Vorrichtung gemäß Anspruch 1, die Scandaten
von einem intraoralen Scanner (100) empfängt und die Scandaten an eine Datenverarbeitungsvorrichtung (300) überträgt, die Scandaten verarbeitet, die durch den intraoralen Scanner erhalten werden, wobei das Betriebsverfahren Folgendes umfasst:
Erfassen eines Kommunikationszustands mit dem intraoralen Scanner über ein drahtloses Kommunikationsmodul (210) der elektronischen Vorrichtung und mechanisches Steuern von mindestens einem von Folgendem:
einer Neigungsrichtung einer Antenne (214), gemäß dem erfassten Kommunikationszustand, unter Verwendung einer motorisierten Neigungsstütze (244), deren Neigung durch eine Signalstärke gesteuert wird,
einer Drehung der Antenne, gemäß dem erfassten Kommunikationszustand, unter Verwendung einer motorisierten Drehstütze (243), deren Drehung durch die Signalstärke gesteuert wird,
einer Neigungsrichtung eines Gehäuses der elektronischen Vorrichtung, in der die Antenne installiert ist, gemäß dem erfassten Kommunikationszustand, unter Verwendung eines motorisierten Scharniers (800), dessen Neigung durch die Signalstärke gesteuert wird, oder
einer Drehung des Gehäuses der elektronischen Vorrichtung, in der die Antenne installiert ist, gemäß dem erfassten Kommunikationszustand, unter Verwendung einer motorisierten Drehplatte (270), deren Drehung durch die Signalstärke gesteuert wird.

11. Betriebsverfahren nach Anspruch 10, wobei das drahtlose Kommunikationsmodul (210) der elektronischen Vorrichtung ein zweites Kommunikationsmodul (212) beinhaltet, das in einem 60-GHz-Signalsende-/-empfangsband in Betrieb ist, wobei das Betriebsverfahren ferner Folgendes umfasst:
Erhalten mindestens einer von der Neigungsrichtung der Antenne (214) oder der Neigungsrichtung des Gehäuses der elektronischen Vorrichtung, in der die Antenne installiert ist, für die eine Signalstärke, die von dem zweiten Kommunikationsmodul empfangen wird, einen Schwellenwert überschreitet, und
mechanisches Steuern mindestens einer von der Antenne oder dem Gehäuse der elektronischen Vorrichtung gemäß mindestens einer von der erhaltenen Neigungsrichtung der Antenne oder der erhaltenen Neigungsrichtung des Gehäuses.

12. Betriebsverfahren nach Anspruch 10, ferner umfassend:
Erfassen eines Kommunikationszustands mit dem intraoralen Scanner (100); und
Bereitstellen einer Benutzerrückkopplungsführung, um eine Position oder eine Richtung von mindestens einem von dem intraoralen Scanner oder der elektronischen Vorrichtung als Reaktion auf den erfassten Kommunikationszustand einzustellen.

13. Betriebsverfahren nach Anspruch 12, ferner umfassend: Bereitstellen der Benutzerrückkopplungsführung, um die Position oder die Richtung von mindestens einem von dem intraoralen Scanner (100) oder der elektronischen Vorrichtung einzustellen, wenn der erfasste Kommunikationszustand angibt, dass eine Kommunikation in einem 2,4-GHz-Bereich möglich ist und dass eine Kommunikation in einem 60-GHz-Bereich unmöglich ist.

14. Betriebsverfahren nach Anspruch 13, wobei die elektronische Vorrichtung ferner eine Kühleinrichtung beinhaltet, um die Wärmeerzeugung der elektronischen Vorrichtung zu beseitigen,
wobei das Betriebsverfahren ferner Folgendes umfasst:
Erfassen eines Betriebszustands des intraoralen Scanners (100) und Steuern der Kühleinrichtung der elektronischen Vorrichtung gemäß dem erfassten Betriebszustand des intraoralen Scanners.

## Revendications

1. Dispositif électronique qui reçoit des données de numérisation provenant d'un scanner intra-buccal (100) et transmet les données de numérisation à un dispositif de traitement de données (300) traitant les données de numérisation, le dispositif électronique (200) comprenant :
un module de communication sans fil (210) ;
une mémoire comprenant une ou plusieurs instructions ; et
un processeur (230) configuré pour exécuter la ou les instructions pour commander le module de communication sans fil afin de recevoir les données de numérisation transmises du scanner intra-buccal, et commander les données de numérisation reçues pour être traitées et transmises au dispositif de traitement de données,
dans lequel le module de communication sans fil comprend une antenne (214) pour recevoir les données de numérisation transmises du scanner intra-buccal,
dans lequel l'antenne est disposée ou commandée pour avoir au moins l'un d'un emplacement ou d'une direction approprié(e) pour recevoir les données de numérisation, et
dans lequel le processeur est en outre configuré pour exécuter la ou les instructions pour :
détecter un état de communication avec le scanner intra-buccal par l'intermédiaire du module de communication sans fil du dispositif électronique, et
commander mécaniquement au moins l'une parmi :
une direction d'inclinaison de l'antenne, en fonction de l'état de communication détecté, à l'aide d'un support d'inclinaison motorisé (244) dont l'inclinaison est commandée par une intensité de signal,
une rotation de l'antenne, en fonction de l'état de communication détecté, à l'aide d'un support de rotation motorisé (243) dont la rotation est commandée par l'intensité de signal,
une direction d'inclinaison d'un boîtier du dispositif électronique où l'antenne est installée, en fonction de l'état de communication détecté, à l'aide d'une charnière motorisée (800) dont l'inclinaison est commandée par l'intensité de signal, ou
une rotation du boîtier du dispositif électronique où l'antenne est installée, en fonction de l'état de communication détecté, à l'aide d'une plaque de rotation motorisée (270) dont la rotation est commandée par l'intensité de signal.

2. Dispositif électronique de la revendication 1, dans lequel le boîtier du dispositif électronique comprend la charnière motorisée (800) pour commander une inclinaison de l'antenne (214).

3. Dispositif électronique de la revendication 1, dans lequel :
l'antenne (214) est disposée de manière à présenter une inclinaison sur un côté du boîtier du dispositif électronique, et
le côté du boîtier du dispositif électronique est installé de manière à présenter une inclinaison correspondant à l'inclinaison de l'antenne.

4. Dispositif électronique de la revendication 1, dans lequel :
le module de communication sans fil (210) comprend un second module de communication (212) fonctionnant dans une bande de transmission/réception de signal de 60 GHz, et
le processeur (230) est configuré pour exécuter la ou les instructions pour :
obtenir au moins l'une de la direction d'inclinaison de l'antenne (214) ou de la direction d'inclinaison du boîtier du dispositif électronique où l'antenne est installée pour laquelle une intensité de signal reçu provenant du second module de communication dépasse un seuil, et
commander mécaniquement au moins l'un de l'antenne ou du boîtier du dispositif électronique en fonction d'au moins l'une de la direction d'inclinaison obtenue de l'antenne ou de la direction d'inclinaison obtenue du boîtier.

5. Dispositif électronique de la revendication 1, dans lequel le processeur (230) est configuré pour exécuter la ou les instructions pour :
détecter un état de communication avec le scanner intra-buccal (100) ; et
fournir une rétroaction utilisateur guidant l'ajustement d'un emplacement ou d'une direction d'au moins l'un du scanner intra-buccal ou du dispositif électronique en réponse à l'état de communication détecté.

6. Dispositif électronique de la revendication 5, dans lequel le processeur (230) est configuré pour exécuter la ou les instructions afin de fournir la rétroaction utilisateur guidant l'ajustement de l'emplacement ou de la direction d'au moins l'un du scanner intra-buccal (100) ou du dispositif électronique lorsque l'état de communication détecté indique que la communication est possible dans une plage de 2,4 GHz et que la communication est impossible dans une plage de 60 GHz.

7. Dispositif électronique de la revendication 6, dans lequel le processeur (230) est configuré pour exécuter la ou les instructions pour :
détecter un état de fonctionnement du scanner intra-buccal (100), et
commander un moyen de refroidissement du dispositif électronique en fonction de l'état de fonctionnement détecté du scanner intra-buccal.

8. Dispositif électronique de la revendication 5, dans lequel le processeur (230) est configuré pour exécuter la ou les instructions pour :
commander un moyen de refroidissement du dispositif électronique pour qu'il fonctionne lorsqu'un état de fonctionnement du scanner intra-buccal (100) indique que le scanner intra-buccal fonctionne, et
commander le moyen de refroidissement du dispositif électronique pour qu'il ne fonctionne pas lorsque l'état de fonctionnement du scanner intra-buccal indique que le scanner intra-buccal est en attente de fonctionnement.

9. Dispositif électronique de la revendication 7, dans lequel le processeur (230) est configuré pour exécuter la ou les instructions afin de détecter l'état de fonctionnement du scanner intra-buccal (100) en recevant des informations sur l'état de fonctionnement du scanner intra-buccal provenant du scanner intra-buccal.

10. Procédé de fonctionnement d'un dispositif électronique selon la revendication 1 qui reçoit des données de numérisation provenant d'un scanner intra-buccal (100) et transmet les données de numérisation à un dispositif de traitement de données (300) traitant des données de numérisation obtenues par le scanner intra-buccal, le procédé de fonctionnement comprenant :
la détection d'un état de communication avec le scanner intra-buccal par l'intermédiaire d'un module de communication sans fil (210) du dispositif électronique, et
la commande mécanique d'au moins l'une parmi :
une direction d'inclinaison d'une antenne (214), en fonction de l'état de communication détecté, à l'aide d'un support d'inclinaison motorisé (244) dont l'inclinaison est commandée par une intensité de signal,
une rotation de l'antenne, en fonction de l'état de communication détecté, à l'aide d'un support de rotation motorisé (243) dont la rotation est commandée par l'intensité de signal,
une direction d'inclinaison d'un boîtier du dispositif électronique où l'antenne est installée, en fonction de l'état de communication détecté, à l'aide d'une charnière motorisée (800) dont l'inclinaison est commandée par l'intensité de signal, ou
une rotation du boîtier du dispositif électronique où l'antenne est installée, en fonction de l'état de communication détecté, à l'aide d'une plaque de rotation motorisée (270) dont la rotation est commandée par l'intensité de signal.

11. Procédé de fonctionnement de la revendication 10, dans lequel le module de communication sans fil (210) du dispositif électronique comprend un second module de communication (212) fonctionnant dans une bande de transmission/réception de signal de 60 GHz,
ledit procédé de fonctionnement comprenant en outre :
l'obtention d'au moins l'une de la direction d'inclinaison de l'antenne(214) ou de la direction d'inclinaison du boîtier du dispositif électronique où l'antenne est installée pour laquelle une intensité de signal reçu provenant du second module de communication dépasse un seuil, et
la commande mécanique d'au moins l'un de l'antenne ou du boîtier du dispositif électronique selon au moins l'une de la direction d'inclinaison obtenue de l'antenne ou de la direction d'inclinaison obtenue du boîtier.

12. Procédé de fonctionnement de la revendication 10, comprenant en outre :
la détection d'un état de communication avec le scanner intra-buccal (100) ; et
la fourniture d'une rétroaction utilisateur guidant l'ajustement d'un emplacement ou d'une direction d'au moins l'un du scanner intra-buccal ou du dispositif électronique en réponse à l'état de communication détecté.

13. Procédé de fonctionnement de la revendication 12, comprenant en outre : la fourniture de la rétroaction utilisateur guidant l'ajustement de l'emplacement ou de la direction d'au moins l'un du scanner intra-buccal (100) ou du dispositif électronique lorsque l'état de communication détecté indique que la communication est possible dans une plage de 2,4 GHz et que la communication est impossible dans une plage de 60 GHz.

14. Procédé de fonctionnement de la revendication 13, dans lequel le dispositif électronique comprend en outre un moyen de refroidissement pour éliminer la génération de chaleur du dispositif électronique,
ledit procédé de fonctionnement comprenant en outre :
la détection d'un état de fonctionnement du scanner intra-buccal (100), et
la commande du moyen de refroidissement du dispositif électronique en fonction de l'état de fonctionnement détecté du scanner intra-buccal.
